# EUROPEAN PATENT SPECIFICATION

(11) **EP 3 539 527 B1**
(45) Date of publication and mention of the grant of the patent: **17.11.2021**
(21) Application number: 19159516.4
(22) Date of filing: 26.02.2019
(51) Int. Cl.: A61H 1/02, A61H 3/00, A61H 3/04

(54) **CONTROLLING POSITION OF WEARABLE ASSISTIVE DEVICE DEPENDING ON OPERATION MODE**
STEUERUNG DER POSITION EINER ASSISTIERENDEN WEARABLE-VORRICHTUNG IN ABHÄNGIGKEIT DES BETRIEBSMODUS
CONTRÔLE DE LA POSITION D'UN DISPOSITIF D'ASSISTANCE PORTABLE SELON LE MODE DE FONCTIONNEMENT

(30) Priority: 15.03.2018 KR 20180030469; 12.06.2018 KR 20180067660; 12.09.2018 US 201862730399 P; 12.09.2018 US 201862730400 P; 12.09.2018 US 201862730412 P; 12.09.2018 US 201862730420 P
(43) Date of publication of application: 18.09.2019
(73) Proprietor: LG Electronics Inc., Seoul 07336 (KR)
(72) Inventor: NAM, Bohyun, 08592 Seoul (KR); PARK, Kyu Tae, 08592 Seoul (KR); SON, Jung Kyu, 08592 Seoul (KR); YU, Seonil, 08592 Seoul (KR); LEE, Wonjun, 08592 Seoul (KR)
(74) Representative: Vossius & Partner Patentanwälte Rechtsanwälte mbB

(56) References cited:
- EP-A1- 3 207 909
- KR-B1- 100 716 597
- KR-B1- 101 788 567

## Description

### BACKGROUND

### 1. Field

This application relates to assistive and/or rehabilitative technology.

### 2. Background

In assistive and/or rehabilitative technology, an assistive device such as a wearable robot, e.g., exoskeleton, may assist or augment a movement of a user. The exoskeleton may be donned on a part of the body and may have a multi-joint skeletal structure to move with a joint movement of the user, and the exoskeleton may further provide an assistive force to the user.

Generally, the exoskeleton may have a motor or driving means to generate the assistive force. The multi-joint structure and frame of the exoskeleton may be made of a metal material, and so the exoskeleton may weigh tens of kilograms or more. For a user to wear such a heavy exoskeleton, an assistant to the user may have to carry or transfer the exoskeleton to the user by using a separate transportation device. When there is no separate transportation device, multiple people may have to carry the exoskeleton. In addition, if the assistant carries an exoskeleton by herself, she may be injured due to the heavy weight of the exoskeleton.

In order to solve the above-mentioned problem, a conventional walking assistive apparatus as disclosed in Korean Patent No. 10-1433284 (FIGS. 1 and 2) and US Patent Application No. 2016-0045382 (FIGS. 3 and 4) may be used. Hereinafter, the walking assistive apparatus will be described with reference to the above-mentioned prior documents.

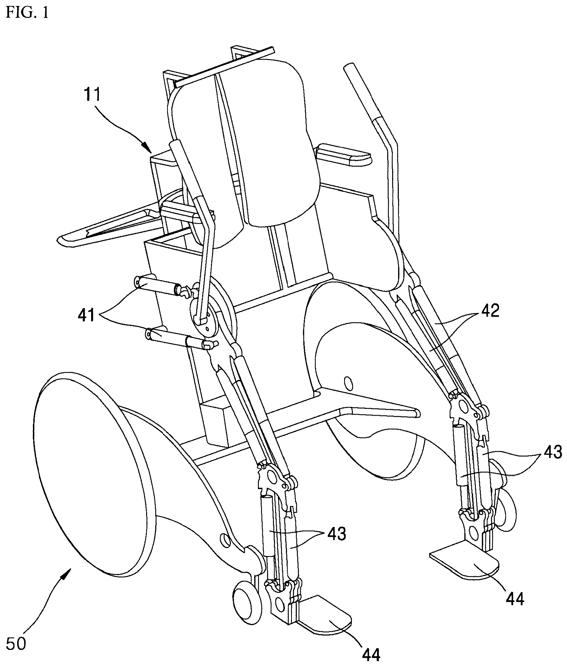

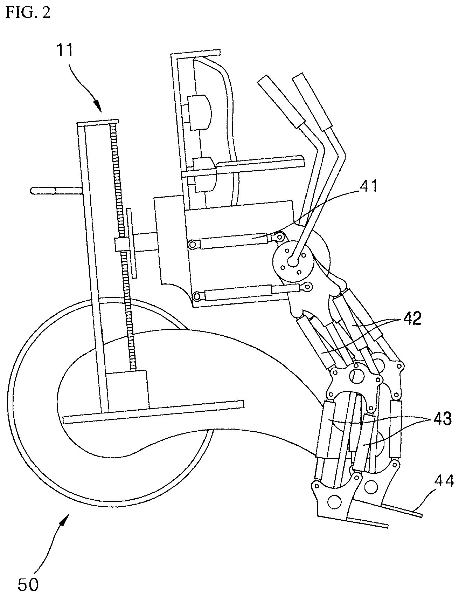

FIGS. 1 and 2 are views showing a conventional walking assistive apparatus in Korean Patent No. 10-1433284. Referring to FIGS. 1 and 2, the walking assistive apparatus may include a frame 11, walking assistive units or shafts 41, 42, 43, 44, and a wheel 50.

The frame 11 may move the walking assistive shafts 41, 42, 43, and 44 on the wheel 50 upward and downward. The legs of the user may be set with the walking assistive shafts 41, 42, 43, and 44. The wheel 50 may move the walking assistive units 41, 42, 43, and 44 forward, rearward, leftward, and rightward.

The height of the walking assistive shafts 41, 42, 43, 44 may be adjusted for rehabilitation training in a state in which the user wears the walking assistive shafts 41, 42, 43, and 44. The height of the walking assistive shafts 41, 42, 43, and 44 may be changed by manually operating the frame unit 11. When the user wears the walking assistive shafts 41, 42, 43, and 44, the user may not be able to adjust the height of the walking assistive shafts 41, 42, 43, and 44 by himself. Therefore, the user may require an additional person to help the user.

When adjusting the frame 11 upward and downward, the walking assistive shafts 41, 42, 43, and 44 may collide with the ground. When frequent collisions occur, a durability of a drive system and joints between the walking assistive shifts 41, 42, 43, and 44 may be reduced.

Further, the walking assistive apparatus may be difficult to put on, and so a user may have trouble preparing to wear the walking assistive apparatus. A typical user may also be weak, making it even harder to don the walking assistive apparatus. Thus, the user may need the help of a number of assistants to prepare to use the walking assistant apparatus.

When the user wears the walking assistive apparatus in a sitting state by using a separate chair, the user or his assistants may have to manually set a posture and placement of the conventional walking assistive apparatus such that it corresponds to a shape or position of a chair. Setting a posture or placement of the walking assistive apparatus may be difficult and may also require a number of assistants to help.

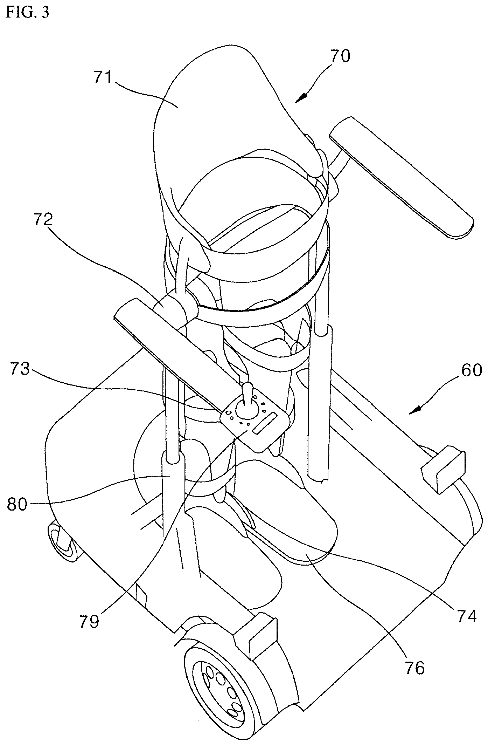

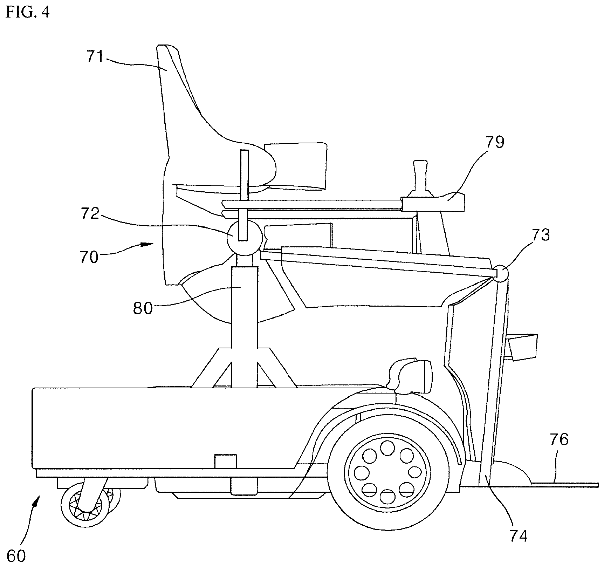

FIGS. 3 and 4 are views showing a conventional standing wheelchair in US Patent Application No. 2016-0045382. Referring to FIGS. 3 and 4, the conventional standing wheelchair may include a base 60, a harness 70, and a lifting unit or shaft 80.

In the standing wheelchair, the base 60 may provide a wheel on a lower side. The harness 70 may support a body of a user. The lifting shaft 80 may be arranged on the base 60. The lifting shaft 80 may adjust the height of the harness 70. The harness unit 70 may include a chair 70, a hip joint 72, a knee joint 73, and an ankle joint 74. Depending on the height change of the harness 70, a position and an angle of each component of the harness 70 may be changed.

The hip joint 72 and the lifting shaft 80 may provide a driving means and may be operated. Alternatively, the knee joint 73 and the ankle joint 74 may move due to a movement of other components. Thus, the conventional standing wheelchair may provide an assistive force to aid a user in standing. However, it does not provide a joint assistive force to aid the user in walking. Further, in the conventional standing wheelchair, the harness 70 that secures the body of the user and the lifting shaft 80 may be formed integrally. Therefore, a range of motion of the user wearing the harness 70 may be very limited.

In the standing wheelchair, a load of the harness 70 may be continuously applied to the base 60 when the standing wheelchair is stored for a long time without being used. Due to the continuous weight and stress applied on the base 60, a durability of the standing wheelchair may be reduced, increasing a probability of damage.

EP 3 207 909 A1 relates to a supporting structure for assisting a disabled person in travelling comprising two pairs of legs and four wheels, each arranged at a lower end of a respective one of the legs. Each leg comprises an upper segment and a lower segment and optionally a retractable leg extension segment for contacting ground instead of the corresponding wheel.

### BRIEF DESCRIPTION OF THE DRAWINGS

The embodiments will be described in detail with reference to the following drawings in which like reference numerals refer to like elements wherein:
FIGS. 1 and 2 may be views illustrating a conventional walking assistive apparatus according to the prior art;
FIGS. 3 and 4 may be views illustrating a conventional standing wheelchair according to the prior art;

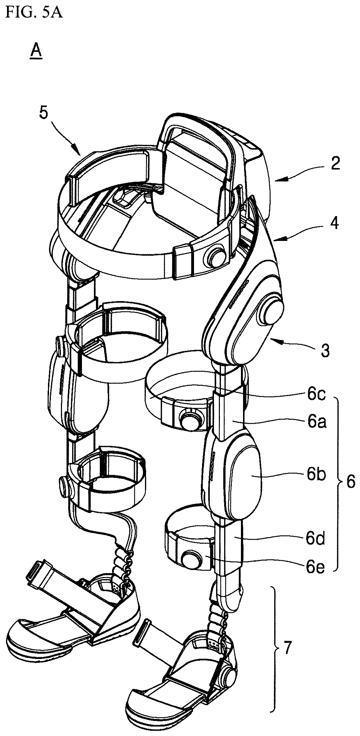

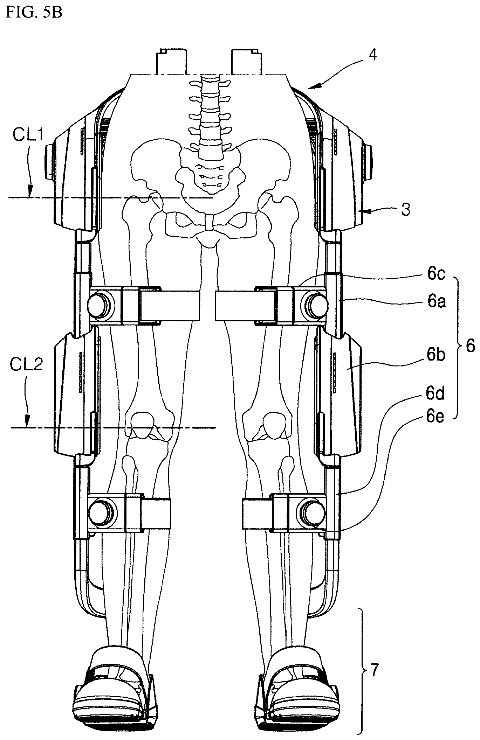

FIGs. 5A and 5B are views showing an exoskeleton in accordance with an exemplary embodiment;

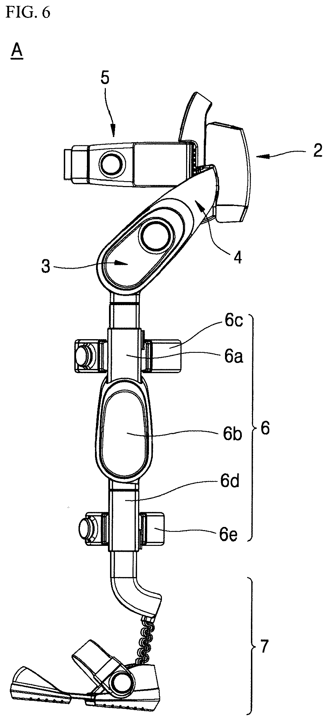

FIG. 6 is a side view of an exoskeleton according to FIG. 5;

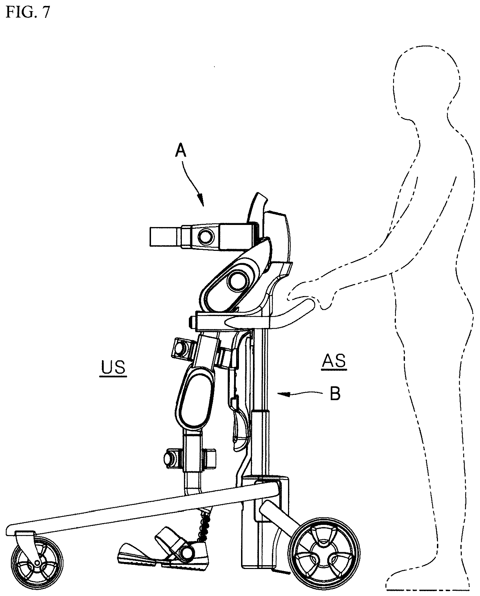

FIG. 7 is a view showing 'a moving mode' of an adaptive assistive and/or rehabilitative device according to an exemplary embodiment;

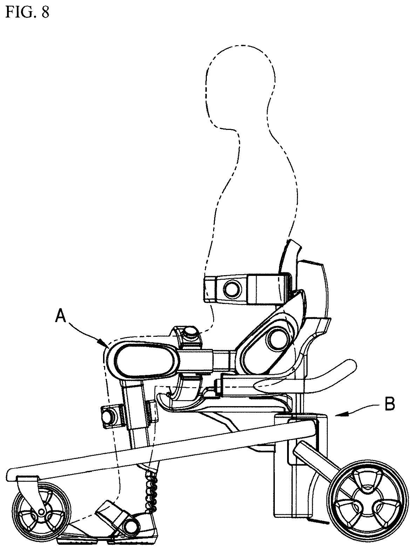

FIG. 8 is a view showing 'a wearing mode' of an adaptive assistive and/or rehabilitative device according to an exemplary embodiment;

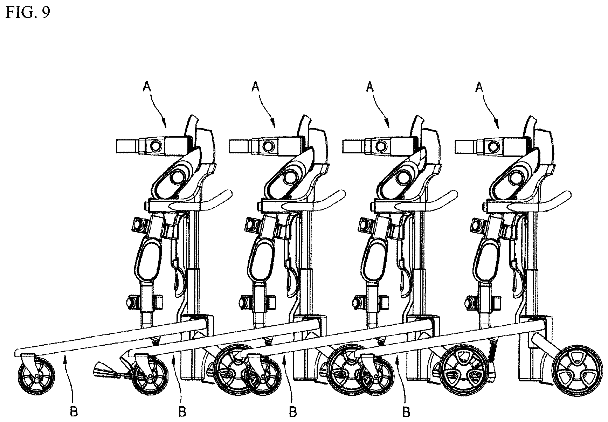

FIG. 9 is a view showing 'a storage mode' of an adaptive assistive and/or rehabilitative device according to an exemplary embodiment;

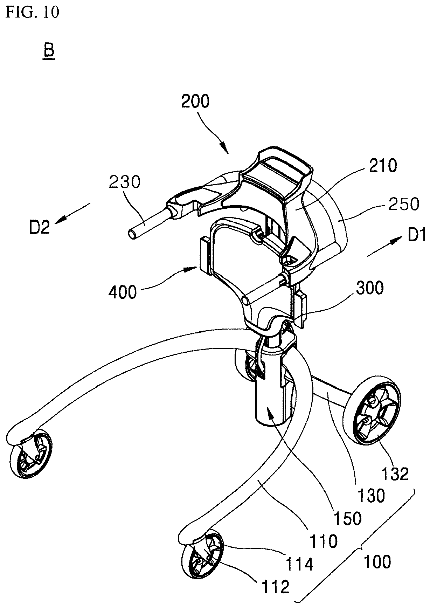

FIG. 10 is a perspective view of an adaptive assistive and/or rehabilitative device according to an exemplary embodiment;

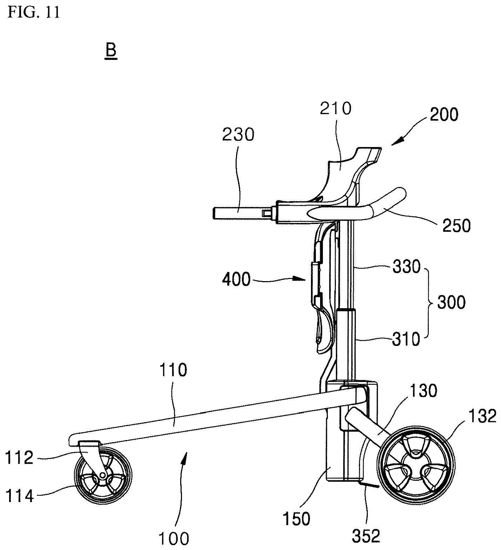

FIG. 11 is a side view of the exoskeleton support of FIG. 10;

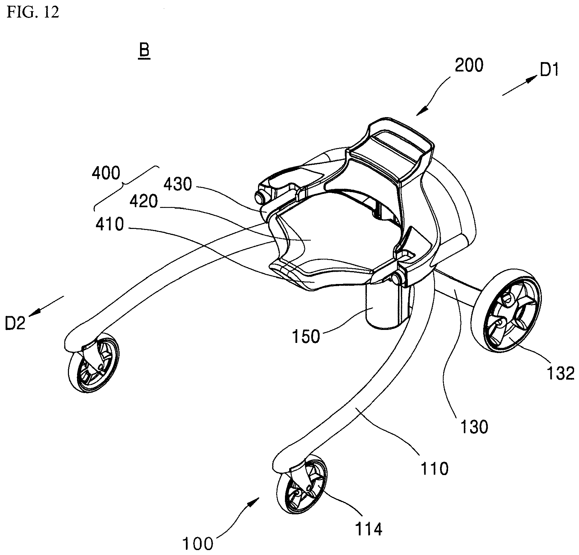

FIG. 12 is a perspective view of a chair state or seated state of an adaptive assistive and/or rehabilitative device according to an exemplary embodiment;

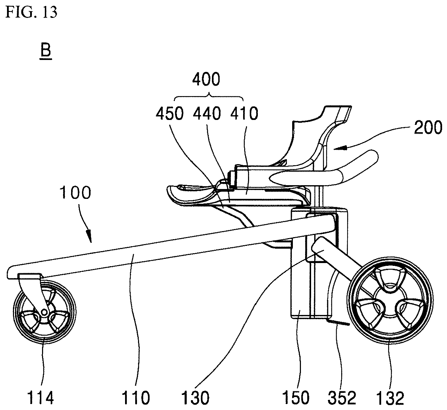

FIG. 13 is a side view of the an adaptive assistive and/or rehabilitative device of FIG. 12;

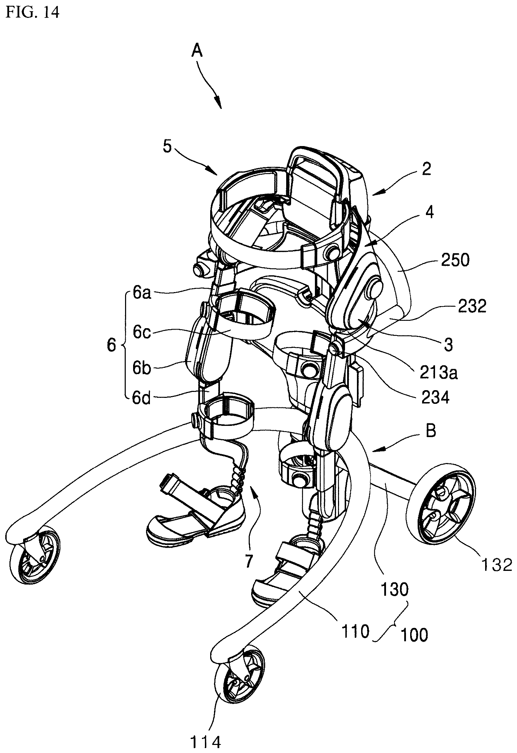

FIG. 14 is a view showing a state in which an exoskeleton is supported on an adaptive assistive and/or rehabilitative device according to an exemplary embodiment;

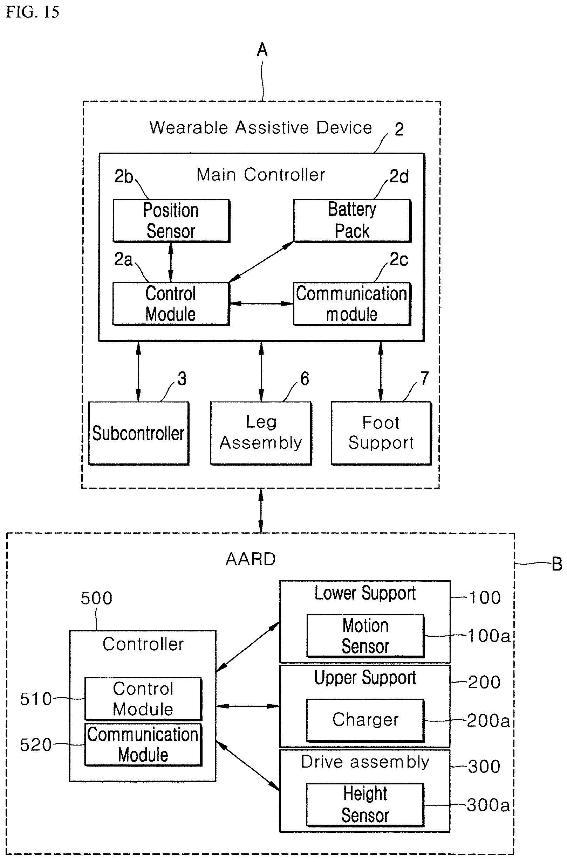

FIG. 15 is a block diagram indicating a mutual relationship between an exoskeleton and an adaptive assistive and/or rehabilitative device according to an exemplary embodiment;

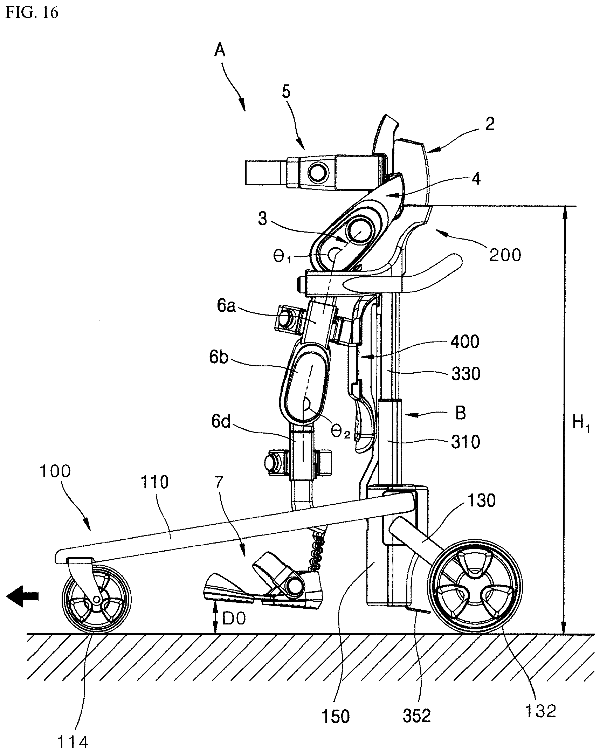

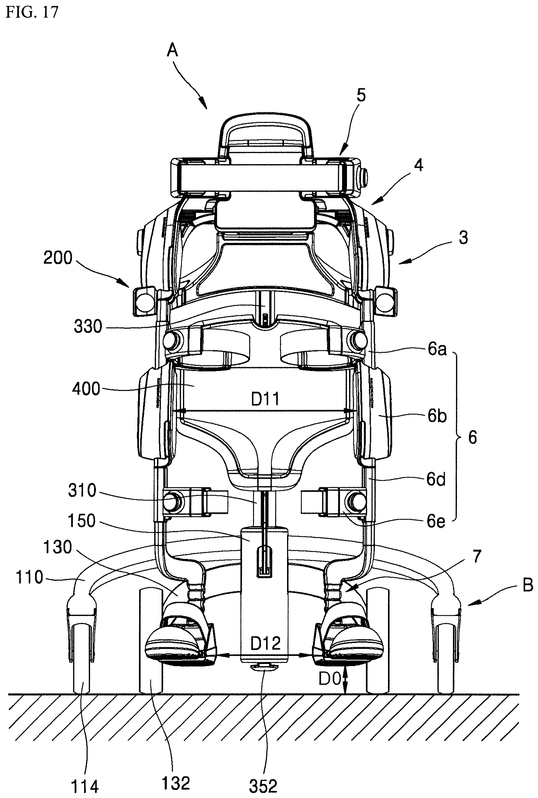

FIGS. 16 and 17 are views exemplifying 'a moving mode' of an exoskeleton according to an exemplary embodiment;

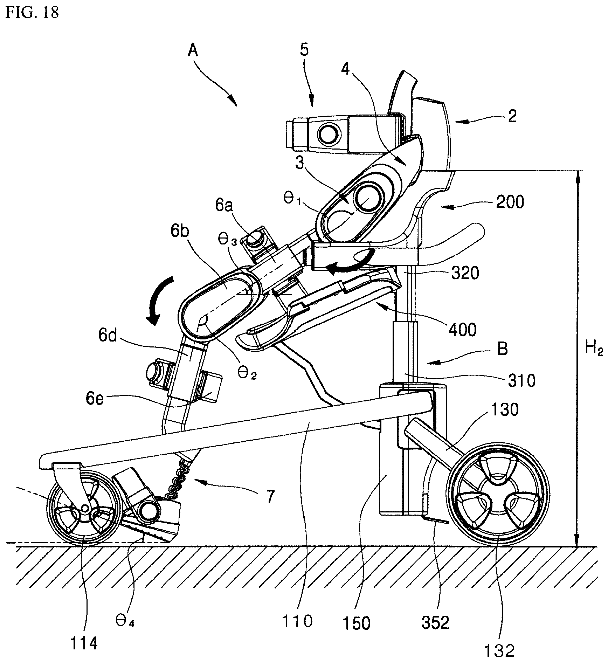

FIG. 18 is a view illustrating a method of controlling an exoskeleton based on a height change of an adaptive assistive and/or rehabilitative device;

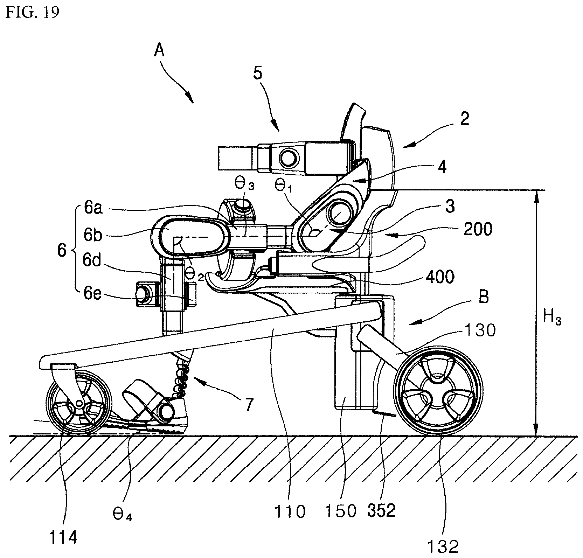

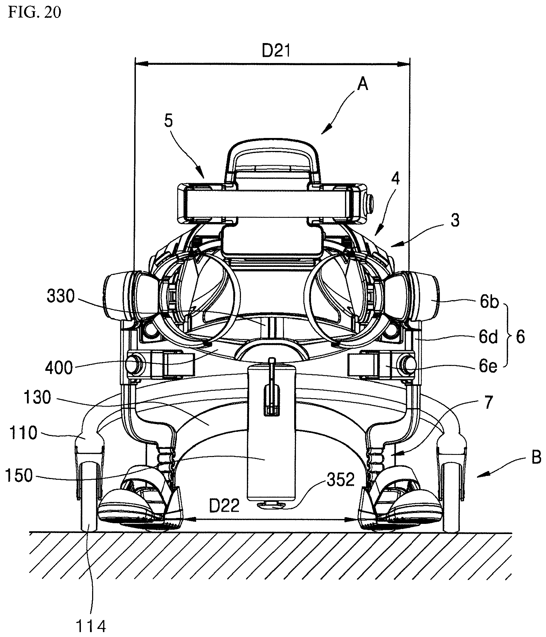

FIGS. 19 and 20 are views illustrating 'a wearing mode' of n exoskeleton according to an exemplary embodiment; and

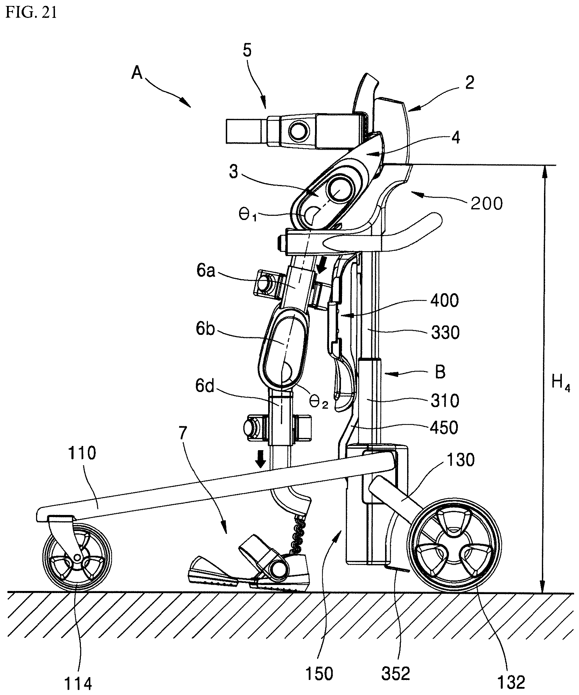

FIGS. 21 to 23 are views illustrating 'a storage mode' of an exoskeleton according to an exemplary embodiment.

### DETAILED DESCRIPTION

The present invention is defined by the independent claims. The dependent claims describe embodiments thereof. In describing this specification, when a detailed description of the known related technology may obscure the gist of this disclosure unnecessarily, the detailed description will be omitted. In this specification, 'a user' means a person who wears a wearable assistive device such as an exoskeleton. Further, 'an assistant' means a person who helps a user. The assistant may help in preparing the user to use the exoskeleton by transporting it from a storage location to a rehabilitation location, for example. The assistant may further help the user to put on the exoskeleton, and may help in a rehabilitation training while the user wears the exoskeleton.

In the present specification, 'an assistive force' may be an external force additionally provided to complement a user's natural motion or strength. The assistive force may be provided in a specific direction to generate an external force using an electric motor, hydraulic pump, or an actuator. The assistive force may be a rotational force that moves the exoskeleton at its joints to correspond with a natural movement of the user.

Referring to FIGS. 5-6, when the user wears a wearable assistive device such as a wearable robot A, e.g., an exoskeleton, on a lower body for walking, the exoskeleton A may assist or add to a lower body power or strength of the user. The exoskeleton A may include a lumbar/back frame 2, a main control unit or main controller 2' housed in the lumbar/back frame 2 (FIG. 15), an actuated hip joint 3, a sub control unit or subcontroller 3' housed near the actuated hip joint (FIG. 15), a main frame 4, a waist/pelvic frame 5, a leg or leg assembly 6, and a foot assembly or foot support 7.

When the user wears the exoskeleton A, the lumbar/back frame 2 may be provided at a rear of the user. The main controller 2' may adjust the width of the main frame 4 to correspond to a body size of the user. The lumbar/back frame 2 may also house a battery pack therein.

The waist/pelvic frame 5 may be coupled to the lumbar/back frame 2. The waist/pelvic frame 5 may be worn on the waist or a pelvis of the user to support a waist of the user. The waist/pelvic frame 5 may include a belt or strap which may be adjustable in length via a one-touch dial or knob. The belt may secure the waist of the user to the exoskeleton A.

The lumbar/back frame 2 may be coupled to the main frame 4. The main frame 4 may have a form that covers a first side, e.g., a left side, of a pelvis of the user to a second side, e.g., a right side, of the user. The main frame 4 may be formed of an approximately 'U'-shape or may be shaped to fit on the user's body. The main frame 4 may include a first extension having a first end and a second extension having a second end. The first and second extensions may extend downward along the hips or pelvis, e.g., ilium, of the user. The first and second extensions may extend from first and second sides of the main frame 4, respectively.

The actuated hip joint 3 may be arranged on first and second extensions of the main frame 4. The subcontroller 3' may be provided on or at the actuated hip joint 3 and may generate a first assistive force. The first assistive force may be a force that assists a strength and movement of the user at the hip joint. The subcontroller 3' may include a rotary dial or knob. The user may adjust the magnitude of the first assistive force via the rotary dial. A driving means, e.g., an actuator or motor, that may provide the first assistive force may be provided in the actuated hip joint 3. The leg 6 may be coupled to a lower end of the actuated hip joint 3. The actuator may be a hydraulic actuator, a pneumatic actuator, or an electrical actuator.

There may be a pair of legs 6, which may be secured to both legs of the user, respectively. Each leg 6 may include an upper leg frame 6a, an actuated joint 6b, a lower leg frame 6d, and leg belts or leg straps 6c and 6e.

The upper leg frame 6a may support and secure to a thigh of the user via the leg belt 6c. A first end of the upper leg frame 6a may be connected to the main frame 4, and a second end of the upper leg frame 6a may be connected to the lower leg frame 6d. A first angle between the upper leg frame 6a and the main frame 4 (Θ1 in Figures 18-19) may be adjusted via the actuated hip joint 3 and/or the subcontroller 3' such that the hip joint can rotate around axis CL1 (FIG. 5B). Θ1 may also be referred to as a hip joint angle.

The upper leg frame 6a may also be extended outward, e.g., toward a left or right side by a hip joint structure (not shown) of the main frame 4. The user wearing the exoskeleton A may therefore extend his or her legs out to his right side or left side from a midline of his body in a frontal plane of motion.

The actuated joint 6b may include a driving means or leg drive, e.g., a motor or actuator (e.g., a hydraulic actuator, a pneumatic actuator, or an electrical actuator) that provides a second assistive force. The second assistive force may be a force that assists a strength or movement of the user at the knee. The actuated joint 6b may be arranged between the upper leg frame 6a and the lower leg frame 6d. Based on the actuated joint 6b, the upper leg frame 6a and the lower leg frame 6d may move to correspond to a natural movement of a knee joint of the user. The leg drive and/or the actuated joint 6b may adjust a second angle between the upper leg frame 6a and the lower leg frame 6d (angle Θ2 in Figures 18 and 19) so that the knee may bend around axis CL2 (FIG. 5B). Θ2 may also be referred to as a knee joint angle.

The lower leg frame 6d may support and be secured to the calf via leg belt 6e. The leg belts 6a and 6e may include a belt having a length adjustable via a rotary dial or knob.

The foot support 7 may be coupled to a lower end of the lower leg frame 6d. The foot support 7 may be secured to and support a foot or a shoe of the user via a strap. The foot support 7 may be worn on a bare foot, sock, or a shoe. Hereinafter, for convenience of description, an example where the shoes or feet of the user are secured to the foot support 7 will be described.

The foot support 7 may be formed in a shape corresponding to that of the shoes of the user. A length of the foot support 7 may be adjusted at a base of the foot support 7. The foot support 7 may include at least one pressure sensor (not shown). Data measured in the pressure sensor may be transmitted to the main controller 2'. Based on received data, the main controller 2' may determine whether the foot support 7 is in contact with a floor surface or a ground. Based on that determination, the main controller 2' may control an operation of the subcontroller 3' and the leg 6. Alternatively, the main controller 2' may control the actuated hip joint 3 directly instead of controlling the subcontroller 3' to control the actuated hip joint 3. A detailed description thereof will be described later with reference to FIG. 15.

The exoskeleton A may be supported on an adaptive assistive and/or rehabilitative device (hereinafter, AARD) B, which may serve multiple functions such as storing A, charging, and transporting the exoskeleton A. The user may further use the AARD B to sit and to support himself when he walks. Hereinafter, each operation mode of the AARD B will be described. FIG. 7 shows 'a moving mode' of an AARD in accordance with an exemplary embodiment. FIG. 8 shows 'a wearing mode' of an AARD in accordance with an exemplary embodiment. FIG. 9 shows 'a storage mode' of an AARD in accordance with an exemplary embodiment.

Referring to FIG. 7, an exoskeleton A may be supported on an AARD B. When the AARD B is in a moveable state or the moving mode, the AARD B may be used as a walker to support the user while walking, or may be used to transport the exoskeleton A. The AARD B and the exoskeleton A may form an assistive rehabilitative system, or ARS. The ARS may be in a walker state or transport state when the AARD B is in a moving mode. When the exoskeleton A is supported on the AARD B while the AARD B is in a moving mode, then the ARS may be in a transport state. The AARD B may be in a standing state when its operation mode is the moving mode or storage mode, and the AARD B may be in a seated state when its operation mode is the wearing mode.

In the moving mode, wheels 114 and 132 of the AARD B may spin or turn, or may not be fixed by a brake. The exoskeleton A may be supported on the AARD B in an upright state in a transport state of the ARS when the AARD B is in the moving mode. The exoskeleton A, primarily at the foot support 7, may be maintained in a state spaced apart from the ground so as not to drag on the ground. Such a configuration may prevent damage to the foot support 7 during transportation.

The exoskeleton A may be supported on a user side (US) of the AARD B, and an assistant to the user may move the AARD B while holding an assistant handle or transport handle provided on an assistant side (AS) of the AARD B (see FIG. 7). The user side of the AARD B may be the side on which a user sits or walks while using the AARD B, while the assistant side of the AARD B may be on a side opposite to the user side. The assistant may stand on the assistant side of the AARD B when she transports the AARD B. The assistant may move the exoskeleton A, which may weigh up to tens of kilograms, by applying a small force to the AARD B at the transport handle. A detailed description of an operation of the exoskeleton A and the AARD B in the moving mode will be described later with reference to FIGS. 16 and 17.

Referring to FIG. 8, an AARD B may serve as a chair so that the user may wear an exoskeleton A in a seated state or chair state of the ARS. Hereinafter, a state in which the AARD B may be or serve as a chair may be defined as a wearing mode or 'donning mode'. When an operation mode of the AARD B is switched from the moving mode to the wearing mode, a seat or chair assembly of the AARD B may be switched from a standing state to a seated or chair state. In a process of switching from the moving mode to the wearing mode, the height of the AARD B may be lowered. Depending on a height change of the AARD B, a posture of the exoskeleton A may be automatically controlled. A detailed description of an operation of the exoskeleton A and the AARD B in the wearing mode will be described later with reference to FIGS. 19 and 20.

Referring to FIG. 9 in a state in which the exoskeleton A may be supported, the AARD B may be stored for a predetermined storage time or more. Hereinafter, a state in which a movement of the AARD B may be stopped for a predetermined storage time or more may be defined as 'a storage mode' or 'charging mode'.

In the storage mode, a first exoskeleton A₁ may be supported on a first AARD B₁ in a standing posture. When a plurality of exoskeleton supports Bₙ are closely attached or overlapped while a plurality of exoskeletons Aₙ are supported, a part of the first AARD B₁ may be overlapped with a second AARD B₂. The first AARD B₁ may be horizontally stacked with a second AARD B₂ to a degree which it may not interfere with the first or second exoskeletons A₁ and A₂. As a result, it may be possible to store a number of exoskeletons Aₙ in a small space, improving a space utility of the AARD B .

When there are more than two exoskeletons A supported on AARDs B, they may be arranged such that the AARDs B overlap with each other in a state where the exoskeletons A do not touch each other. In the storage mode, an outer sole of the foot support 7 for each exoskeleton A may touch the ground at the foot support 7. As a result, a load applied to the AARD B may be reduced and a use life of the AARD B and the exoskeleton A may be preserved.A detailed description of an operation of the exoskeleton A and the AARD B in the storage mode will be described in detail with reference to FIGS. 21 to 23.

FIG. 10 shows an AARD B in accordance with an exemplary embodiment. FIG. 11 shows a side view of the AARD B of FIG. 10. Hereinafter, for convenience, a first direction D1 may be defined as a user direction or a walking direction, and a second direction D2 may be defined as an assistant direction or a transfer direction. With reference to Figures 10 and 11, the AARD B may include a lower assembly or lower support 100, an upper assembly or upper support 200, a driving unit or drive assembly 300, and a seat or chair assembly 400.

The lower support 100 may support an overall weight of the AARD B, including that of the upper support 200. The lower support 100 may have a plurality of wheels 114 and 132. A brake may be provided in the plurality of wheels 114 and 132. When the brake is operated, the wheels 114 and 132 may be stopped in a parked state. On the contrary, when the brake is not operated, the wheels 114 and 132 may be in a moveable state.

Depending on whether the wheels 114 and 132 are stopped, an operation mode of the AARD B may be changed. For example, when the wheels 114 and 132 are in a fixed or parked state, the operation mode of the AARD B may be a wearing or donning mode or a storage mode, and the ARS may be in a storage state or chair state. When the wheels 114 and 132 are in a moveable state, the operation mode of the AARD B may be a moving mode, and the ARS may be in the transport state or walker state.

The lower support 100 may include a motion sensor (100a of FIG. 15). The motion sensor 100a may sense a rotational operation of the wheels 114 and 132, and may also sense an operation of the brake. Based on the rotational operation of the wheels 114 and 132, the motion sensor 100a may produce a motion signal. Based on the operation of the brake, the motion sensor 100a may also produce a brake signal or braking signal. The motion signal and braking signal may be used to determine the operation mode of the AARD B.

The exoskeleton A may be substantially supported in the upper support 200. The upper support 200 may include a main supporting frame or main frame 210, a user or walker handle 230, and the transport handle 250. The main frame 210 may form an appearance of the upper support 200.

The walker handle 230 may be arranged on the user side of the main frame 210. The transport handle 250 may be arranged on the assistant side of the main frame 210. The upper support 200 may include a charging assembly or charger (200a of FIG. 15) therein. The charger 200a may wirelessly provide power to the exoskeleton A or wirelessly charge the exoskeleton A.

The drive assembly 300 may adjust the height of the upper support 200. The drive assembly 300 may include a lower pipe or shaft 310, an upper pipe or shaft 330, and a driving means or drive, e.g., hydraulic (or pneumatic) cylinder or motor and gear set. The drive of the drive assembly 300 may provide a driving force to raise or lower the upper shaft 330. A pedal 352 may operate as a switch to control the drive of the drive assembly 300. The user may operate or stop the drive by pushing the pedal 352.

The lower shaft 310 may be coupled to the lower housing 150. The upper shaft 330 may be inserted into the lower shaft 310. Alternatively, the lower shaft 310 may be inserted into the upper shaft 330. Depending on such coupling relationship, the cross-sectional area or size of the lower shaft 310 and the upper shaft 330 may be varied. For example, if the upper and lower shafts 330 and 310 are cylindrical, an outer diameter of the upper shaft 330 may correspond to an inner diameter of the lower shaft 310 when the upper shaft 330 is inserted into the lower shaft 310. The upper and lower shafts 330 and 310 may have a cylindrical shape or square tube shape, but shapes of the upper and lower shafts 310 and 330 are not limited thereto. For example, the lower shaft 310 may have a cylindrical shape, while the upper shaft 330 may have a cylindrical shape with a flat edge or plate edge.

The drive assembly 300 may include a drive sensor or height sensor 300a. The height sensor 300a may sense an operation of the drive to generate height information. The height information may include information on a driving direction, such as ascending and descending, and a driving amount, such as a force of the drive or a time of an operation of the drive. The height information may be used to determine the operation mode of the AARD B. A controller 500 (FIG. 15) of the AARD B may receive the height information from the height sensor 300a. Based on the received height information, the controller 500 may calculate a height of the AARD B.

The controller 500 may compare the calculated height to a predetermined reference height to determine the operation mode of the AARD B. For example, when the calculated height of the AARD B is higher than the predetermined reference height, the controller 500 may determine the AARD B is in a moving mode or a storage mode. When the calculated height is lower than the predetermined reference height, the controller 500 may determine the AARD B to be in the wearing mode.

Further, the controller 500 may transmit height information to the exoskeleton A. Like the AARD B, the exoskeleton A may determine the operation mode of the AARD B by using received height information. The content thereof will be described in detail with reference to FIG. 15.

Additionally, the controller 500 may receive an ascending control signal or ascension signal or a descending control signal or descension signal from the exoskeleton, in addition to controlling a posture of the exoskeleton A. The user may select or control the ascending and/or descending control signals, or the main controller 2' may generate the ascending and/or descending control signals. For example, a user may input a command to the main controller 2 to fix or lower the drive assembly 300. If the main controller 2' senses that a predetermined amount of time has passed since the exoskeleton A has been used, the main controller 2' may generate a descension control signal to lower the drive assembly 300 so that the exoskeleton A contacts the ground, in addition to controlling a posture of the exoskeleton A.

Based on the ascending control signal and the descending control signal, the controller 500 may operate, or raise and/or lower, the drive of the drive assembly 300. For example, when the controller 500 receives the ascending control signal, the controller 500 may raise the upper shaft 330 to increase the height of the AARD B. When the controller 500 receives the descending control signal, the controller 500 may lower the upper shaft 330 to decrease the height of the AARD B.

As shown in FIGS. 12 and 13, the chair assembly 400 may be rotatably coupled to the upper shaft 330. The chair assembly 400 may include a seat frame 410, a seat 420, a sub supporter or side support 430, a link frame 440, and a support link or seat link 450. The seat frame 410 may form an appearance of a seat of a chair. A rear end of the seat frame 410 may be wider than that of a front end. In other words, a width of the seat frame 410 may recede away from the drive assembly 400. Typically, when a user sits on a chair, his legs may naturally open slightly outward. Therefore, the seat frame 410 may be smaller further away from the driving assembly 300 so that the legs of the user can open outward without interfering with the seat.

The seat 420 may be provided on an upper surface of the seat frame 410. The seat 420 may be formed integrally with the seat frame 410. Alternatively, the seat 420 maybe separately manufactured to be coupled to the seat frame 410. A shape of the seat 420 may correspond to a shape of the seat frame 410, or may correspond to a shape of a buttocks of the user when the user sits in the seat 420.

The side support 430 may be provided on a side of the seat 420. The chair 400 may include two supports 430, each provided on a separate side of the seat 420. The side support 430 may support a part of the exoskeleton A at a section of the leg 6. The link frame 440 may be coupled to a lower surface of the seat frame 410. The seat link 450 may be coupled to a link bracket, which may couple to the link frame 440. The seat link 450 may rotatably connect the chair assembly 400 and the lower housing 150. As a result, the seat link 450 may aid in a movement of the seat 420 when the AARD B transitions between standing and seated states.

When the seat 420 is unfolded in a seated state of the AARD B, the seat frame 410 may be perpendicular to the upper shaft 330 and parallel to the ground. In a state in which the seat 420 may be folded in a standing state of the AARD B, the seat frame 410 may be parallel to the upper shaft 330. In the wearing or donning mode of the AARD B, the chair assembly 400 and the AARD B may be in a chair or seated state. In the moving mode or the storage mode of the AARD B, the chair assembly 400 and the AARD B may be in a standing state.

FIG. 14 shows a state in which an exoskeleton A may be supported on an AARD B in accordance with an exemplary embodiment. FIG. 15 is a block diagram showing an exoskeleton A and an AARD B in accordance with an exemplary embodiment. Referring to FIGS. 14 and 15, the exoskeleton A may be supported on the AARD B.

The main controller 2' may primarily control a posture of the exoskeleton A, while the controller 500 may primarily control a height and movement of the AARD B. The main controller 2' and the controller 500 may communicate with each other to optimize a position and posture of the exoskeleton A when it is supported on the AARD B. The main controller 2' of the exoskeleton A may include a control portion or control module 2a, a position sensor 2b, a communication module 2c, and a power source module or a battery pack 2d. The control module 2a may control a position and an angle of the leg 6 by controlling an operation of the subcontroller 3' in the actuated hip joint 3 and a leg drive in the actuated joint 6b. The control module 2a may alternatively control the motor or drive in the actuated hip joint 3 directly.

The control module 2a may control the subcontroller 3' (which may also serve as a slave controller) and thus an operation of the actuated hip joint 3 to change a first angle θ1 (Fig. 16) between the main frame 4 and the upper leg frame 6a. Further, the control module 2a may control an operation of the actuated joint 6b to change a second angle Θ2 (FIG. 16) between the upper leg frame 6a and the lower leg frame 6d. The control module 2a may receive data from a pressure sensor provided in the foot support 7. Based on the received data, the control module 2a may determine whether the foot support 7 is in contact with the ground.

When the foot support 7 contacts the ground in the moving mode, the control module 2a may control the operation of the actuated hip joint 3 and the actuated joint 6b to space the foot support 7 apart from the ground. When the control module 2a determines from the pressure sensor that the foot support 7 is in contact with the ground, it may adjust the first and second angles Θ1 and Θ2 such that the foot support 7 is lifted from the ground to prevent contact.

The control module 2a may also reduce an overall length of the leg 6. The upper leg frame 6a and the lower leg frame 6d may be formed of a plurality of frame members, respectively. The control module 2a may increase an overlapping length of the plurality of frame members so that the length of the upper leg frame 6a and a length of the lower leg frame 6d may be reduced (or increased), respectively. As a result, when the overall length of the leg 6 is reduced, the foot support 7 may be spaced apart from the ground. The user may directly adjust the upper and lower leg frames 6a and 6d to adjust the overlapping length. Alternatively, there may be a driving means, motor, or actuator (hydraulic, pneumatic, or electric) provided in the upper and lower leg frames 6a and 6d to adjust the overlapping length.

Further, by controlling the subcontroller 3' and/or the actuated hip joint 3, the control module 2a may adjust the first angle θ1 (Fig. 16) between the main frame 4 and the upper leg frame 6a. Similarly, by controlling the actuated joint 6b, the control module 2a may adjust the second angle θ2 (Fig. 16) between the upper leg frame 6a and the lower leg frame 6d. By adjusting the first angle θ1 and the second angle θ2, the foot support 7 may be spaced apart from the ground. A combination of the first angle θ1 and the second angle θ2 may be variously configured to space the foot support 7 apart from the ground.

The control module 2a may generate an ascending control signal to increase the height of the AARD B. Then, a generated ascending control signal may be transmitted to the controller 500 of the AARD B. When the controller 500 receives the ascending control signal, the controller 500 may operate the drive assembly 300 to increase an overall height of the AARD B. Thus, the exoskeleton A may be spaced apart from the ground. Accordingly, when the AARD B is moved, the exoskeleton A may not collide with the ground.

On the other hand, when the foot support 7 is spaced apart from the ground in the wearing mode or the storage mode, the operation of the actuated hip joint 3 and the actuated joint 6b may be controlled so that the foot support 7 contacts the ground. The control module 2a may increase the overall length of the leg 6 by reducing the overlapping length of the plurality of frame members that comprise the upper leg frame 6a and/or the lower leg 6d so that the length of each of the upper leg frame 6a and the lower leg frame 6d may be increased. As a result, the overall length of the leg 6 may be increased, and the foot support 7 may contact the ground.

Further, by controlling the subcontroller 3 and thus the actuated hip joint 3, the control module 2a may adjust the first angle θ1 between the main frame 4 and the upper leg frame 6a. Similarly, by controlling the actuated hip joint 6b, the control module 2a may adjust the second angle θ2 between the upper leg frame 6a and the lower leg frame 6d. A combination of the first angle θ1 and the second angle θ2 may be variously controlled and configured to allow the foot support 7 to contact the ground.

In addition to controlling the exoskeleton A to space the foot support 7 apart from the ground, the control module 2a may also control the AARD B. The control module 2a may generate a descending control signal that decreases the height of the AARD B. The generated descending control signal may be transmitted to the controller 500 of the AARD B. When the controller 500 receives the descending control signal, the controller 500 may operate the drive assembly 300 to reduce the overall height of the AARD B by lowering the height of the upper shaft 330. Accordingly, the foot support 7 may contact the ground, and a load of the exoskeleton A applied to the AARD B may be dispersed. Since the foot support 7 may maintain a state in contact with the ground, the user may easily fix his or her shoes to the foot support 7. As a result, a convenience of the exoskeleton A may be improved in simplifying the application and/or dressing process.

The position sensor 2b may sense position information of the exoskeleton A. The position sensor 2b may include various modules capable of sensing position information. For example, the position sensor 2b may include a Global Positioning System (GPS) or an Inertial Measurement Unit (IMU). This may be merely one example, and various types of position measurement modules may be included in the position sensor 2b. Data measured in the position sensor 2b may be transmitted to the control module 2a. Based on data measured in the position sensor 2b, the control module 2a may calculate the height of the main controller 2'.

Using the height of the main controller 2' of the exoskeleton A, the control sensor 2a may determine an operation mode of the AARD B. In order to determine the operation mode of the AARD B, the control module 2a may use a predetermined exoskeleton reference height. For example, when the calculated height of the main controller 2' is higher than the predetermined exoskeleton reference height, the control module 2a may determine the operation mode of the AARD B as 'a moving mode' or 'a storage mode'. On the contrary, when the calculated height of the main controller 2' is lower than the predetermined exoskeleton reference height, the control module 2a may determine the operation mode of the AARD B as 'a wearing mode'.

The communication module 2c may exchange data with the AARD B. The communication module 2c may receive a motion signal from a communication module 520 in the controller 500 of the AARD B. The motion signal may be generated in the motion sensor 100a provided in the lower support 100. The motion sensor 100a may sense a movement of the wheels 114 and 132 provided in the lower support 100. When the wheels 114 and 132 move, the motion signal may be activated.

When a motion signal is received, the control module 2a may determine the operation mode of the AARD B as 'a moving mode'. On the contrary, when the received motion signal is inactivated, the control module 2a may determine the operation mode of the AARD B as 'a wearing mode' or 'a storage mode'.

The communication module 2c may receive the braking signal from the communication module 520 of the controller 500 of the AARD B. Similarly, the braking signal may be generated in the motion sensor 100a provided in the lower support 100. The motion sensor 100a may sense whether the brake is applied or operated. Further, when a predetermined time elapses, the brake may automatically be applied. When the brake is applied, the braking signal may be activated.

When the braking signal is inactive or not received, the control module 2a may determine the operation mode of the AARD B as 'a moving mode'. On the contrary, when the braking signal is activated (that is, in an unmovable state), the control module 2a may determine the operation mode of the AARD B as 'the wearing mode' or 'a storage mode'. Thus an operation mode of the AARD B may be determined based on the motion signal, the braking signal, or a combination thereof.

Further, the communication module 2c may receive height information from the communication module 520 of the AARD B. Height information may be generated in the height sensor 300a provided in the drive assembly 300. The height sensor 300a may sense a movement of the above-mentioned drive assembly 300. The height sensor 300a may, for example, include a laser distance sensor to measure a distance to the ground. Height information generated in the height sensor 300a may include information on an operation direction and an operation amount of the drive in the drive assembly 300. By using received height information, the control module 2a may calculate the height of the AARD B and compare the calculated height sensed in the height sensor to a predetermined drive reference height.

When the calculated height of the AARD B is higher than the predetermined AARD reference height, the control module 2a may determine the operation mode of the AARD B as 'a moving mode' or 'a storage mode'. On the contrary, when the calculated height of the AARD B is lower than the predetermined AARD reference height, the control module 2a may determine the operation mode of the AARD B as 'a wearing mode'.

The power source or battery pack 2d may receive power from a charger 200a of the AARD B. The battery pack 2d may receive power wirelessly from the charger 200a. The provided power may be stored in a battery pack of the main controller 2.

Based on an intensity of a power signal received from the charger 200a by the battery pack 2d, the control module 2a may determine whether the exoskeleton A is supported on the AARD B. In addition, based on an intensity of a communication signal received in the communication module 2c, the control module 2a may determine whether the exoskeleton A is supported. On an assumption that the exoskeleton A may be supported on the AARD B, the control module 2a may perform a control operation depending on the operation mode.

The AARD B may include the lower support 100, the upper support 200, the drive or drive assembly 300, and the controller 500. Since a content of the lower support 100, the upper support 200, and the driving unit 300 may have been described in detail above, a repeated content may be omitted.

The controller 500 may include the control module 510 and the communication module 520. The control module 510 may control an operation of each component included in the AARD B. For example, the control module 510 may control the operation of the drive provided in the drive assembly 300, and may control an operation of the brakes in the wheels 114 and 132.

The communication module 520 may exchange data with the communication module 2c of the exoskeleton A. The communication module 520 may transmit received data to the control module 510. The control module 510 may control the operation of the drive assembly 300 based on received data. For example, when the ascending control signal is received in the communication module 520, the control module 510 may operate the drive provided in the drive assembly 300. As a result, the upper support 200 may be raised and the exoskeleton A supported on the AARD B may be spaced apart from the ground.

On the other hand, when the descending control signal is received in the communication module 520, the control module 510 may operate the drive provided in the drive 300. As a result, the upper support 200 may be lowered, and the exoskeleton A supported on the AARD B come in contact with the ground.

Referring to FIGS. 16 and 17, in 'the moving mode', the height H of the AARD B may be kept higher than the predetermined AARD reference height at a first height or standing height H1. Further, the wheels 114 and 132 of the AARD B may not be fixed or parked in the moving mode. Accordingly, in the moving mode, the AARD B may maintain a movable state. In order for the exoskeleton A to move without colliding with or dragging on the floor in a state in which it is supported on the AARD B, the foot assembly 7 of the exoskeleton A may be maintained spaced apart from the floor.

The main controller 2' may calculate a first height or a standing height H1 of the AARD B in a state in which the exoskeleton A is supported on the AARD B. The height H of the AARD B may be calculated based on position information sensed in the position sensor 2b. Alternatively, the height H may be calculated based on height information sensed in the height sensor 300a. The main controller 2' may then determine whether the H may be at a height H1 higher than the predetermined AARD reference height by comparing the calculated height H with the above-mentioned AARD reference height.

Based on a movement signal or a brake signal received from the motion sensor 100a, the main controller 2' may determines whether the AARD B may be in a movable state. For example, when the movement signal is activated or the brake signal is inactivated, the AARD B may be in a movable state. When the height H is at a height H1 calculated to be higher than the predetermined reference height and the AARD B is in a movable state, the main controller 2' may determine that the operation mode of the AARD B is 'a moving mode'.

Based on data measured in the pressure sensor provided in a lower surface of a foot support 7, the main controller 2' may determine whether the foot support 7 is in contact with the ground. The operation of the leg 6 and the actuated hip joint 3 may be controlled so that the foot support 7 is no longer spaced apart from the ground if the foot support 7 currently contacts the ground. The main controller 2' may reduce the length of the leg 6 so that the exoskeleton A may be spaced apart from the ground.

As previously described, the upper leg frame 6a and the lower leg frame 6d of the leg 6 may be formed of a plurality of frame members, respectively. The length of the upper leg frame 6a and the lower leg frame 6d may be reduced by increasing the overlapping length of the plurality of frame members, which is controlled by the main controller 2'. As a result, the overall length of the leg 6 may be reduced, and the foot support 7 may be spaced apart from the ground by a predetermined distance D0.

Further, by controlling the subcontroller 3' and/or the actuated hip joint 3, the main controller 2' may adjust the first angle or hip joint angle θ1 (FIG. 18) between the main frame 4 and the upper leg frame 6a. Similarly, by controlling the actuated joint 6b, the main controller 2' may adjust the second angle or knee joint angle θ2 between the upper leg frame 6a and the lower leg frame 6d. A combination of the first angle θ1 and the second angle θ2 may be variously configured to separate the foot support 7 from the ground.

The main controller 2' may generate an ascending control signal to increase the height of the AARD B. Then, a generated ascending control signal may be transmitted to a controller 500 of the AARD B. When receiving the ascending control signal, the controller 500 may operate the drive assembly 300. As a result, the height H may be increased, and the exoskeleton A may be spaced apart from the ground by predetermined distance D0.

The controller 500 of the AARD B may receive data from the pressure sensor provided on the lower surface of the foot support 7 from the exoskeleton A. Based on the motion signal or the braking signal measured in the motion sensor 100a, and based on height information measured in the height sensor 300a, the controller 500 may determine whether the current operation mode of the AARD B is the moving mode.

When data from the pressure sensor indicates that the foot support 7 is in contact with the ground in the moving mode, the controller 500 may operate the drive to increase the height H to a height H1 greater than the predetermined reference height, and space the foot support 7 apart from the ground by the predetermined distance D0.

As an example, during a storage or charging state, the AARD B may be at a height that is slightly less than a maximum height of the AARD B such that the chair assembly 400 may be slightly unfolded or protruded. The exoskeleton A may be stored on the AARD B even though the chair assembly 400 is partially unfolded, and the foot assembly 7 may contact the ground. When the AARD B is switched to the moving mode so that the exoskeleton A can be transported, the controller 500 may operate the drive of the AARD B to increase the height of the AARD B such that the foot assembly 7 no longer contacts the ground. The AARD B may be increased to a maximum height of the AARD B where the chair assembly 400 is completely folded.

When the exoskeleton A is moved in a state in which it is supported on the AARD B, the exoskeleton A may be maintained to be spaced apart from the ground. Therefore, collisions between the exoskeleton A and the ground may be reduced. Accordingly, a usable life of the exoskeleton A may be prolonged, along with an operation reliability. Further, required maintenance and reparation costs of the exoskeleton A may be reduced.

FIG. 18 illustrates a method of controlling an exoskeleton A depending on the height change of an AARD B in accordance with an exemplary embodiment. When an AARD B switches from the moving mode or the storage mode to the wearing mode, the overall height of the AARD B may be reduced. Referring to FIG. 18, when a height of the AARD B is reduced within a transformation height range H2, the main controller 2' may control an operation of a subcontroller 3'.

The first angle θ1 between the main frame 4 and the upper leg frame 6a may be reduced. Accordingly, a third angle θ3 formed between the upper leg frame 6a and the ground may be also reduced. The second angle θ2 between the upper leg frame 6a and the lower leg frame 6d may be unchanged while the first angle θ1 is reduced. Therefore, a fourth angle θ4 formed between a bottom surface of the foot support 7 and the ground may be increased as the foot support 7 is lifted further away from the ground. The foot support 7 may thus be maintained spaced apart from the ground while the AARD B is still transitioning from the moving mode to the wearing mode.

In another example, while the first angle θ1 is reduced, the second angle θ2 may be reduced. However, when the second angle θ2 changes more quickly than the first angle θ1, the exoskeleton A may collide with the ground. Therefore, a rate of change in the second angle θ2 may be set to be smaller than a rate of change in the first angle θ1. In other words, the second angle θ2 may change at the same rate or less than a rate of change of the first angle θ1.

When the height H of the AARD B is continuously reduced within the transformation height range H2, the main controller 2' may drive the leg drive in the actuated joint 6b. The second angle θ2 between the upper leg frame 6a and the lower leg frame 6d may be reduced. Accordingly, the fourth angle θ4 formed by the bottom surface of the foot support 7 and the ground may also be decreased. When the height H of the AARD B is continuously reduced, the outer sole at the toe of foot support 7 may be maintained spaced apart from the ground. When the AARD B is completely in the wearing mode, which is described later, the exoskeleton A may then be controlled so that the outer sole of the foot support 7 contacts the ground.

Alternatively, the foot support 7 may not contact the ground throughout the entire transition process, but the foot support 7 may still be angled such that the fourth angle θ4 represented an angle from a reference line parallel to the ground from the outer sole at a heel of the foot support 7 and the outer sole at the toe of the foot support 7. The foot support 7 may then first contact the ground at the outer sole at the heel when the AARD B has completely transitioned to the wearing mode.

While the second angle θ2 decreases, the first angle θ1 may be continuously reduced. Therefore, the third angle θ3 and the fourth angle θ4 may diminish at the same time. If the pressure sensor of the foot support 7 senses an impact with the ground while the third angle θ3 and the fourth angle θ4 decrease, the main controller 2' may increase a rate of change of the first angle θ1 and the second angle θ2. This increase in the rate of change may reduce an impact applied to the foot support 7, and/or lift or maintain the foot support 7 away from the ground.

As a result, the exoskeleton A may be maintained in a state spaced apart from the ground. Even when the AARD B changes from the moving mode or the storage mode to the wearing mode, the exoskeleton A may be kept spaced apart from the ground.

FIGS. 19 and 20 illustrate a wearing mode of a wearable exoskeleton in accordance with an exemplary embodiment. Referring to FIGS. 19 and 20, the assistant may move the AARD B to a desired position when the AARD B is in the moveable mode. An assistant may prepare the exoskeleton A for wearing by the user, or may help the user don the exoskeleton A. If the user has an impaired lower body, the assistant may switch the AARD B to 'a wearing mode'.

The assistant may manually apply a force greater than a predetermined force to the upper support 200 from an upper side of the AARD B. The height H of the AARD B may be reduced to a sitting height or a third height H3. Accordingly, the chair assembly 400 may be switched to the chair state or the seated state. Alternatively, the assistant may drive the drive in the drive assembly 300 via a separate switch such as pedal 352 to lower the height H to the sitting height H3 of the AARD B and switch the chair assembly 400 to the seated state, switching the AARD B to 'the wearing mode'.

The main controller 2' may control the subcontroller 3' and/or the actuated hip joint 3 so that the first angle θ1 between the main frame 4 and the upper leg frame 6a decreases in a switching process to the wearing mode. The third angle θ3 between the upper leg frame 6a and the ground may converge to 0 degrees. Therefore, the upper leg frame 6a may be parallel to the ground. The main controller 2' may control the actuated joint 6b so that the second angle θ2 between the upper leg frame 6a and the lower leg frame 6d may be reduced. The fourth angle θ4 formed between the foot support 7 and the ground may be reduced to converge to 0 degrees. Even once the transition to the wearing mode has completed, the foot support 7 may still not completely contact the ground (in other words, the fourth angle θ4 may still have value close to zero but not exactly zero). Alternatively, the foot support 7 may completely contact the ground once the transition to the wearing mode has been completed to further reduce a load applied to the AARD B.

The height H of the AARD B may then be kept at a height H3 lower than a predetermined AARD reference height when the AARD B stops descending such that the AARD B may be completely switched to the wearing mode. In the wearing mode, the AARD B may be maintained in a stopped state. The wheels 114 and 132 of the AARD B may be fixed by the brake, allowing the user to stably don the exoskeleton A.

In the wearing mode, the main controller 2' may calculate the third height H3 of the AARD B. The third height H3 may be calculated based on positional information sensed in the above-mentioned position sensing portion 2b. Alternatively, the third height H3 may be calculated based on height information sensed in the above-mentioned height sensor 300a.

By comparing the calculated third height H3 with the predetermined AARD B reference height, the main controller 2' may determine whether the height H of the AARD is lower than the predetermined AARD B reference height. Further, the main controller 2' may determine whether the AARD B is in a stopped state (or whether it is in a fixed or parked state) based on the motion signal or the braking signal received from the motion sensor 100a. When the motion signal is inactivated, the AARD B may correspond to or be in the stopped or parked state. When the braking signal may be activated, the AARD B may correspond to or be in the stopped or parked state.

When the calculated height H3 is lower than the predetermined reference height and the AARD B may be in a stopped or fixed state, the main controller 2' may determine the operation mode of the AARD B as 'a wearing mode'. Based on data of the pressure sensor provided in the foot support 7, the main controller 2' may determine whether the foot support 7 is in contact with the ground.

In an alternative embodiment, the predetermined AARD B reference height may be referred to as a first predetermined AARD B reference height. The calculated third height H3 may be compared with an optional second predetermined AARD B reference height that is lower than the first predetermined AARD B reference height. The main controller 2' may determine that the AARD B is in the wearing mode if the calculated third height H3 is lower than the optional second predetermined AARD chair height. The main controller 2' may determine that the AARD B is transitioning between the moving mode and the wearing mode if the calculated third height H3 is between the first predetermined AARD B reference height and the optional second predetermined AARD B reference height. The optional second predetermined AARD B reference height may be referred to as a predetermined chair height, while the first predetermined AARD B reference height may be referred to as a predetermined standing height.

When the foot support 7 is spaced apart from the ground, the operation of the leg 6 and the actuated hip joint 3 may be controlled such that the foot support 7 contacts the ground and is no longer spaced apart from the ground. The main controller 2' may increase the length of the leg 6 so that the exoskeleton A may be in contact with the ground. The lengths of the upper leg frame 6a and the lower leg frame 6d of the leg 6 may be increased, respectively. As a result, the overall length of the leg 6 may be increased, and the foot support 7 may be in contact with the ground to reduce the load applied to the AARD B.

By controlling the subcontroller 3' and/or the actuated hip joint 3, the main controller 2' may adjust the first angle θ1 between the main frame 4 and the upper leg frame 6a. Similarly, by controlling the actuated joint 6b, the main controller 2' may adjust the second angle θ2 between the upper leg frame 6a and the lower leg frame 6d. A combination of the first angle θ1 and the second angle θ2 may be variously configured such that the foot support 7 is in contact the ground .

The main controller 2' may generate a descending control signal to reduce the height of the AARD B. Then, the generated descending control signal may be transmitted to the controller 500 of the AARD B. The controller 500 may operate the drive assembly 300 as it receives the descending control signal. As a result, the height H may be reduced, and the exoskeleton A may be in contact with the ground.

Additionally, the controller 500 of the AARD B may receive data of the pressure sensor provided on the lower surface of the foot support 7 from the exoskeleton A. Then, based on the motion signal or the braking signal measured in the motion sensor 100a and height information measured in the height sensor 300a, the controller 500 may determine whether the current operation mode is the wearing mode. For example, when the motion signal is inactivated, the braking signal is activated, and the calculated height H3 is lower than the above-mentioned AARD B reference height, the controller 500 may determine the current state of the AARD B as 'a wearing mode'.

When the foot support 7 does not contact the ground in'the wearing mode, the controller 500 may operate the drive to reduce the height H further such that the foot support 7 contacts the ground. Additionally, in response to a sitting posture of the user, the leg 6 may be controlled to extend outward from the exoskeleton A by a predetermined angle. It may be convenient to don the exoskeleton A when the legs of the user extend slightly outward while the user sits down.

The main frame 4 may have a hip joint structure. Thus, the leg 6 may be extended outward by a predetermined angle at the main frame 4. The hip joint structure of the main frame 4 or the lumbar/back frame 2 may include a motor or actuator (e.g., electric, pneumatic, or hydraulic) to adjust a width of the main frame 4 or to drive an outward or inward movement of the hip joint structure, increasing or decreasing a distance between two legs 6. Alternatively, the hip joint structure of the main frame 4 may be manually driven, and the user or assistant may manually adjust the legs 6 to adjust the distance between the legs 6.

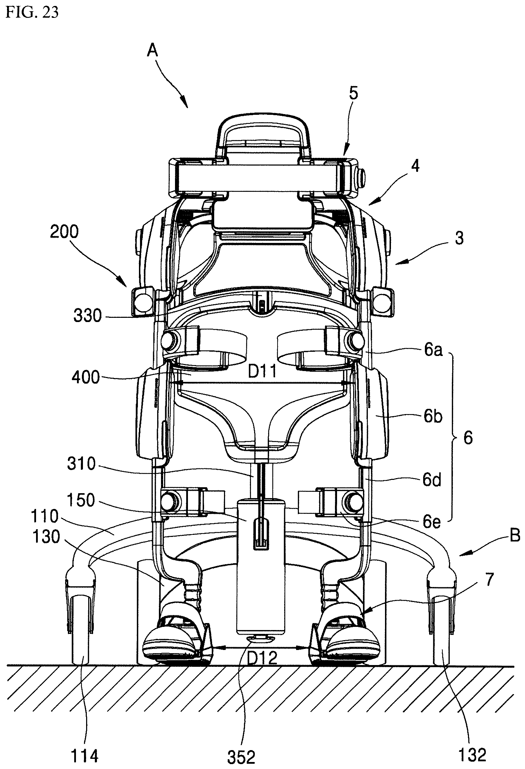

Accordingly, a distance between each leg 6 in a pair of legs 6 (D21 in FIG. 20) in the wearing mode may be wider than a distance between each leg 6 (D11 in FIG. 17 or FIG. 23) in the moving mode or the storage mode. Similarly, a distance (D22 in FIG. 20) between the foot support 7 of each leg 6 in the wearing mod' may be wider than a distance between each foot support 7 (D12 in FIG. 17 or FIG. 23) in 'the moving mode' or storage mode. When the AARD B switches from 'the moving mode' or 'the storage mode' to 'the wearing mode', the posture of the exoskeleton A may be automatically changed so that the user may wear it conveniently.

Accordingly, the user may sit on the chair of the AARD B to wear the exoskeleton A, improving the convenience of the exoskeleton A. Since the posture of the exoskeleton A may be automatically controlled, the user may wear the exoskeleton A without a number of assistants. More users can use the exoskeleton A in a given time frame due to a reduced preparation time, so an efficiency and profitability of the exoskeleton A may be increased and maximized.

FIGS. 21 to 23 illustrate 'a storage mode' of a wearable exoskeleton in accordance with an exemplary embodiment. Referring to FIGS. 21 to 23, the height H of the AARD B may be kept at a fourth height or storage height H4 higher than the predetermined AARD B reference height in the storage mode. In addition, in the storage mode, the wheels 114 and 132 of the AARD B may be maintained in a stopped state for a certain time or predetermined time or more. When the stopped state of the AARD B continues for a predetermined time, the AARD B may be switched from 'a moving mode' to 'a storage mode'.

In 'the storage mode', the exoskeleton A may be controlled to contact the ground, reducing a load applied to the AARD B. In 'the storage mode', the main controller 2 may calculate the fourth height H4, of the AARD B. The fourth height H4 may be calculated based on position information sensed in the position sensor 2b. Alternatively, the fourth height H4 may be calculated based on height information sensed in the height sensor 300a.

The main controller 2' may determine whether the AARD B is in a stopped or parked state for the predetermined time or more based on the motion signal or the braking signal received from the motion sensor 100a. When the height is greater than the predetermined reference height and the wheels 114 and 132 are fixed or stopped, the main controller 2' may determine the operation mode of the AARD B to be 'a storage mode'. Based on data of the pressure sensor, the main controller 2' may determine whether the foot support 7 is in contact with the ground. If the foot support 7 is spaced apart from the ground, an operation of the leg 6 and the actuated hip joint 3 may be controlled such that the foot support 7 contacts the ground.

The main controller 2' may increase the length of the leg 6 so that the exoskeleton A may contact the ground (see FIG. 21). The length of the upper leg frame 6a and the lower leg frame 6d of the leg 6 may be increased, respectively. As a result, the overall length of the leg 6 may be increased, and the foot support 7 may be in contact with the ground.

By controlling the subcontroller 3' and/or the actuated hip joint 3, the main controller 2' may adjust the first angle θ1 between the main frame 4 and the upper leg frame 6a. Similarly, by controlling the actuated joint 6b, the main controller 2' may adjust the second angle θ2 between the upper leg frame 6a and the lower leg frame 6d. A combination of the first angle θ1 and the second angle θ2 may be variously configured such that the foot support 7 may contact the ground.

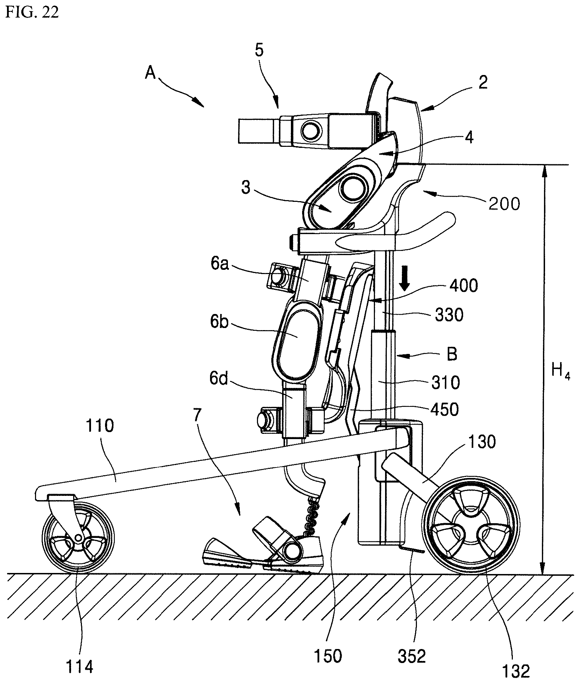

The main controller 2' may generate a descending control signal to reduce the height of the AARD B (see FIG. 22). A generated descending control signal may be transmitted to the controller 500 of the AARD B. The controller 500 may operate the drive assembly 300 to reduce the height H when it receives the descending control signal so that the exoskeleton A may contact the ground.

Based on the motion signal or the braking signal measured in the motion sensor 100a and height information measured in the height sensor 300a, the controller 500 may determine whether the current state is 'the storage mode'. For example, when the motion signal is inactivated or the braking signal is activated and the height H is at a calculated height H4 that is higher than the above-mentioned reference height, the controller 500 may determine the current state as 'a storage mode'.

In the storage mode, when the foot support 7 is spaced apart from the ground, the controller 500 may operate the drive to reduce the height H such that the foot support 7 may contact the ground. The load applied to the AARD B may be reduced when the exoskeleton A is supported on the AARD B while the AARD B is maintained in the stopped or fixed state for a relatively long time. As the load continuously applied to the AARD B is reduced, the useable life of the exoskeleton support may be prolonged. Further, a driving stability and an operation reliability of the AARD B may be improved. Costs required to maintain or repair the AARD B may be reduced.

Since various substitutions, changes, and modifications may be made within the scope that does not deviate the technical idea of this application for those skilled in the art to which this application pertains, this above-mentioned application may be not limited by the above-mentioned embodiments and the accompanying drawings.

Embodiments disclosed herein may control a wearable assistive device or exoskeleton supported on an adaptive assistive and/or rehabilitative device (AARD) or exoskeleton support, in order to correspond to an operation mode of the AARD (i.g., 'a moving mode', 'a wearing mode', or 'a storage mode').

Embodiments disclosed herein may control the exoskeleton so that a foot assembly or foot support, of the exoskeleton may not collide with the ground in 'the moving mode' of the AARD. Embodiments disclosed herein may control the exoskeleton so that a user may wear the exoskeleton in a sitting position or seated state in 'the wearing mode' of the AARD. Embodiments disclosed herein may control the exoskeleton so that a load applied to the AARD may be reduced in 'the storage mode' of the AARD. This load may be reduced when the foot support contacts the ground.

Embodiments disclosed herein are not limited to the above-mentioned objects, and the other objects and the advantages of embodiments disclosed herein which may be not mentioned may be understood by the following description, and more clearly understood by the embodiments of this application. It will be also readily seen that the objects and the advantages of embodiments disclosed herein may be realized by means indicated in the patent claims and a combination thereof.

The exoskeleton according to embodiments disclosed herein may be provided with a main controller that controls the exoskeleton depending on the operation mode ('the moving mode, 'the wearing mode, or 'the storage mode). Based on a height change and a movement of the AARD, the main controller may determine the operation mode. Then, a posture of the exoskeleton may be controlled based on a determined operation mode and whether the foot support contacts the ground. As a result, a convenience of the user may be improved.

The main controller may control an operation of a subcontroller and leg so that the foot support may be spaced apart from the ground in 'the moving mode'. As a result, in a process where the exoskeleton is supported on the AARD and moved, a collision between the exoskeleton and the ground may be prevented.

In addition, the exoskeleton according to embodiments disclosed herein may be provided with a main controller or main control unit that adjusts or controls an angle formed between a leg and the ground, and an angle formed between a foot support, or foot and the ground in 'the wearing mode'. Further, in 'the wearing mode', the distance between two legs may be extended. Thus, the exoskeleton may be controlled so that the user may wear the exoskeleton in a sitting posture.

The main controller may control the operation of the subcontroller or the leg so that the foot support may be in contact with the ground in 'the storage mode'. As a result, when the exoskeleton is supported on the AARD for a long time, the load applied to the AARD may be reduced.

In the exoskeleton, the posture may be controlled depending on the operation mode so that a convenience of the user who uses the exoskeleton may be improved. Further, by preventing an external force from being applied to the exoskeleton, such as a force caused by the exoskeleton dragging on the ground, an operation stability of the exoskeleton may be improved. As a result, a use life or useable life of the exoskeleton may be prolonged. Further, a maintenance cost of the exoskeleton may be reduced, and a reliability of the exoskeleton may be increased.

Since the exoskeleton may be moved in a state of being supported on the AARD, the carrying or transport of the exoskeleton may be made more convenient, as it requires less strength and assistants. The exoskeleton may be maintained in a state spaced apart from the ground when moved. Therefore, the exoskeleton and may not dry on ground in a movement or transport process. Accordingly, the use life of the exoskeleton may be prolonged. Further, the cost of maintenance and repair of the exoskeleton may be reduced.

In the exoskeleton, the posture may be changed so that the user may wear the exoskeleton in a sitting posture in 'the wearing mode'. Accordingly, a convenience of the user may be improved while the user wears the exoskeleton. For example, in a case of a patient or user with an uncomfortable leg, it may be possible for the patient to put on the exoskeleton with minimal movement, and a satisfaction of the wearer may be increased. Further, since a time required to wear and/or use the exoskeleton may be reduced, more users may use the exoskeleton in a given time frame. Therefore, a profitability of an operator who uses or administers the exoskeleton may be improved.

Further, the control of the exoskeleton's posture may reduce the load applied to the AARD. Through a reduction of the load continuously applied to the AARD, the life of the AARD may be prolonged. In addition, a driving stability of the AARD may be improved and a failure rate of the AARD may be reduced.

Embodiments disclosed herein may be implemented as a wearable assistive device comprising a frame, a leg assembly including at least one actuator that provides a rotational force, a foot support coupled to the leg assembly and including a pressure sensor that senses whether the foot support contacts a floor surface, and a controller that receives information from the pressure sensor, and controls the leg assembly and/or the actuator based on information from the pressure sensor.

The leg assembly may include an upper leg frame extending from the frame and a lower leg frame coupled to the upper leg frame, and the controller may control a length of the upper leg frame, a length of the lower leg frame, a hip angle or hip joint angle, a knee angle or knee joint angle, and a foot angle, wherein the hip joint angle is an angle between the frame and the upper leg frame, the knee joint angle is an angle between the upper leg frame and the lower leg frame, and the foot angle is an angle between the foot support and the floor surface.

The wearable assistive device may couple to an adaptive and/or rehabilitative device (AARD), and when the AARD is in motion, the controller may control the lengths of the upper leg frame and the lower leg frame, the hip joint angle, the knee joint angle, and the foot angle such that the foot support does not contact the floor surface while the wearable assistive device is coupled to the AARD.

When the AARD has not been in motion for a predetermined time frame, the controller may control the lengths of the upper leg frame and the lower leg frame, the hip joint angle, the knee joint angle, and the foot angle such that the foot support contacts the floor surface while the wearable assistive device is coupled to the AARD.

The AARD may include a drive assembly that raises or lowers the AARD and includes a height sensor that calculates a height of the AARD, and the controller may control the drive assembly and receive the calculated height. The AARD may further include a wheel that moves the AARD. The wheel may include a motion sensor that senses whether the wheel is moving. When the wheel is moving, the controller may receive a motion signal. A brake may be provided in the wheel to stop the wheel, and the wheel may include a brake sensor that senses whether the brake is applied. When the brake is applied, the controller may receive a braking signal.

The controller may determine that the AARD is in a standing state when the calculated height is greater than a predetermined standing height, and may determine that the AARD is in a seated state when the calculated height is lower than a predetermined chair height.

The controller may determine that the AARD is in a moving mode when the AARD is in a standing state and when the controller receives a motion signal or does not receive a braking signal. The controller may determine that the AARD is in a storage mode when the AARD is in a standing state and when the controller receives a braking signal or does not receive a motion signal for a predetermined amount of time. The controller may determine that the AARD is in a wearing mode when the AARD is in a seated state and when the controller receives a braking signal or does not receive a motion signal

The controller may control the lengths of the upper and lower leg frames, the hip joint angle, the knee joint angle, and the foot angle such that the pressure sensor senses that the foot support does not contact the floor surface in the moving mode, and may controls the lengths of the upper and lower leg frames, the hip joint angle, the knee joint angle, and the foot angle such that the pressure sensor senses that the foot support contacts the floor surface in the storage mode and in the wearing mode. In the wearing mode, the user may sit in a seat of the AARD, secure the frame to a waist, secure the leg assembly to a leg, and secure the foot support to a foot or shoe.

Embodiments disclosed herein may be implemented as a wearable assistive device configured to be supported on an adaptive assistive and/or rehabilitation device (AARD) and configured to receive first and second signals indicating moving and storage modes, respectively, of the AARD, the moving mode being a mode in which the AARD is moveable, and the storage mode being a mode in which the AARD is parked such that movement of the AARD is prevented. The wearable assistive device may include a main frame which secures to a waist or a pelvis, a leg assembly that extends from an end of the main frame, and a main controller. The main controller may control the leg assembly such that the wearable assistive device is spaced apart from a ground upon receiving the first signal indicating the moving mode, and the main controller may control the leg assembly such that the wearable assistive device may contact the ground upon receiving the second signal indicating the storage mode.

The leg assembly may include an upper leg frame connected to an end of the main frame, a lower leg frame connected to an end of the upper leg frame, and an actuated joint that adjusts a knee joint angle, wherein the knee joint angle is an angle between the upper leg frame and the lower leg frame.

The main controller may reduce a length of the upper leg frame or a length of the lower leg frame upon receiving the first signal such that the wearable assistive device is spaced apart from the ground, and may increase the length of the upper leg frame or the length of the lower leg frame upon receiving the second signal such that the wearable assistive device contacts the ground.

The main controller may control the actuated joint to reduce the knee joint angle upon receiving the first signal, and the main controller may control the actuated joint to increase the knee joint angle upon receiving the second signal. The main controller may control an actuated hip joint provided between the upper leg frame and the main frame to reduce a hip joint angle between the upper leg frame and the main frame upon receiving the first signal, and may controls the actuated hip joint to increase the hip joint angle upon receiving the second signal.

The first signal may be a motion signal that is activated when a wheel of the AARD moves, and the second signal may be a braking signal that is activated when a brake is applied to park the AARD.

A foot support provided at an end of the leg assembly may include a pressure sensor that produces a third signal when the foot support contacts the ground, an actuator provided in at least one joint in the leg assembly. A communication module may be provided in the main controller that is configured to receive the first, second, and third signals, and a control module may be provided in the main controller that is configured to control the actuator. When the communication module receives the first signal and the third signal, the control module may control the actuator to reduce an angle at the at least one joint until the control module no longer receives the third signal.

The control module may be configured to control a length of the leg assembly, and may reduce the length of the leg assembly when the communication module receives the first signal and third signal. When the communication module receives the second signal and does not receive the third signal, the control module may control the actuator to increase the angle until the communication module receives the third signal; and when the communication module does not receive any one of the first, second, or third signals for a predetermined time or more, the control module may controls= the actuator to increase the angle until the communication module receives the third signal.

When the main controller receives the first signal and a third signal indicating the wearable assistive device is contacting the ground, the main controller may send an ascending control signal to the AARD to increase the height of the AARD; and when the main controller receives the second signal and does not receive the third signal, the main controller may send a descending control signal to the AARD to reduce the height of the AARD.

Embodiments disclosed herein may be implemented as a wearable assistive device configured to be supported on an adaptive assistive and/or rehabilitation device (AARD) and configured to receive first and second signals indicating transport and donning modes, respectively, of the AARD, the moving mode being a mode in which the AARD is moveable and at a standing height, and the donning mode being a mode in which the AARD is parked at a sitting height such that movement of the AARD is prevented and a seat of the AARD is unfolded. The wearable assistive device may include a main frame configured to support a waist; a leg assembly extending from the main frame and including an actuator provided at a joint; a foot provided at an end of the leg assembly; and a main controller, wherein the main controller controls the leg assembly such that the foot support is spaced apart from a ground upon receiving the first signal indicating the moving mode, and wherein the main controller controls the leg assembly such that the wearable assistive device contacts the ground upon receiving the second signal indicating the donning mode.

The first signal may be a motion signal that is activated when a wheel of the AARD moves or a first height signal that is activated when a height sensor provided in a drive assembly of the AARD senses that the AARD is at the standing state, and the second signal may be a second height signal that is activated when the height sensor of the AARD senses that the AARD is at the sitting height lower than the standing height.

Alternatively, the first signal may be a first height signal that is activated when a position sensor provided in the main controller senses a position that is equal to or greater than a predetermined standing height, and the second signal may be a second height signal that is activated when the position sensor senses a position that is equal to or less than a predetermined sitting height.

The main controller may be configured to control a length of the leg assembly, and may increase the length of the leg assembly upon receiving the second signal so that the foot support contacts the ground.

The leg assembly may include two legs that each extend from the main frame, and the main controller may be configured to control a distance between each leg of the leg assembly, and increases the distance upon receiving the second signal.

A pressure sensor may be provided in the foot assembly that provides a third signal when the foot assembly contacts the ground. The main controller may include a communication module to receive the first, second, and third signals and a control module to control the leg assembly based on the first, second, and third signals.

When the communication module receives the second signal and not the third signal, the control module may send a descending control signal to the AARD to reduce the height of the AARD until the third signal is received; and when the communication module receives the first signal and the second signal, the control module may send an ascending control signal to the AARD to increase the height of the AARD until the third signal is no longer received.

The communication module may receive periodic height signals from a height sensor provided in the AARD indicating height information, and when the communication module receives a first height signal and a second height different from the first height signal, receives the third signal, and does not receive the first or second signals, the control module may control the actuator to reduce the angle in the leg assembly until the third signal is no longer received.

Embodiments disclosed herein may be implemented as a wearable assistive device configured to be supported on an adaptive assistive and/or rehabilitation device (AARD) and configured to receive first and second signals indicating standing and seated states of the AARD, respectively, the standing state being a state in which a height of the AARD is at a first height and the seated state being a state in which the AARD is at a second height lower than the first height; and configured to receive third and fourth signals indicating moving and parked states of the AARD, respectively, the moving state being a state in which the AARD is moveable and the parked state being a state in which movement of the AARD is prevented. The wearable assistive device may comprise a main frame configured to support a waist or pelvis, a leg assembly including a first actuator that provides a rotational force to a first joint, a foot support coupled to the leg assembly and including a pressure sensor that provides a fifth signal when the foot assembly contacts the ground, and a controller. The controller may control the first actuator to adjust a first angle of the first joint such that, upon receiving the first, third, and fifth signals, controls the first actuator to reduce the first angle until the fifth signal is inactive; upon receiving the second signal or upon receiving the first signal together with the fourth signal, controls the first actuator to increase the first angle until the fifth signal is received; and, upon not receiving any of the first, second, third, fourth, or fifth signals for a predetermined time or more, controls the first actuator to increase the first angle until the fifth signal is received.

A second actuator may provide a rotational force to a second joint, and the controller may be configured to control the second actuator to adjust a second angle of the second joint, to control a subcontroller that controls the first actuator, to control a length of the leg assembly, to send signals to the AARD to control a drive assembly that raises and lowers the AARD, and to determine an angle between a heel of the foot support 7 and the ground based on information from the pressure sensor.

It will be understood that when an element or layer may be referred to as being "on" another element or layer, the element or layer may be directly on another element or layer or intervening elements or layers. In contrast, when an element may be referred to as being "directly on" another element or layer, there may be no intervening elements or layers present. As used herein, the term "and/or" may include any and all combinations of one or more of the associated listed items.

It will be understood that, although the terms first, second, third, etc., may be used herein to describe various elements, components, regions, layers and/or sections, these elements, components, regions, layers and/or sections should not be limited by these terms. These terms may be only used to distinguish one element, component, region, layer or section from another region, layer or section. Thus, a first element, component, region, layer or section could be termed a second element, component, region, layer or section without departing from the teachings of the present invention.

Spatially relative terms, such as "lower", "upper" and the like, may be used herein for ease of description to describe the relationship of one element or feature to another element(s) or feature(s) as illustrated in the figures. It will be understood that the spatially relative terms may be intended to encompass different orientations of the device in use or operation, in addition to the orientation depicted in the figures. For example, if the device in the figures may be turned over, elements described as "lower" relative to other elements or features would then be oriented "upper" relative the other elements or features. Thus, the exemplary term "lower" may encompass both an orientation of above and below. The device may be otherwise oriented (rotated 90 degrees or at other orientations) and the spatially relative descriptors used herein interpreted accordingly.

The terminology used herein may be for the purpose of describing particular embodiments only and may be not intended to be limiting of the invention. As used herein, the singular forms "a", "an" and "the" may be intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof.

Embodiments of the disclosure may be described herein with reference to cross-section illustrations that may be schematic illustrations of idealized embodiments (and intermediate structures) of the disclosure. As such, variations from the shapes of the illustrations as a result, for example, of manufacturing techniques and/or tolerances, may be to be expected. Thus, embodiments of the disclosure should not be construed as limited to the particular shapes of regions illustrated herein but may be to include deviations in shapes that result, for example, from manufacturing.

Unless otherwise defined, all terms (including technical and scientific terms) used herein have the same meaning as commonly understood by one of ordinary skill in the art to which this invention belongs. It will be further understood that terms, such as those defined in commonly used dictionaries, should be interpreted as having a meaning that may be consistent with their meaning in the context of the relevant art and will not be interpreted in an idealized or overly formal sense unless expressly so defined herein.

Any reference in this specification to "one embodiment," "an embodiment," "example embodiment," etc., means that a particular feature, structure, or characteristic described in connection with the embodiment may be included in at least one embodiment of the invention. The appearances of such phrases in various places in the specification may be not necessarily all referring to the same embodiment. Further, when a particular feature, structure, or characteristic may be described in connection with any embodiment, it may be submitted that it may be within the purview of one skilled in the art to effect such feature, structure, or characteristic in connection with other ones of the embodiments.

## Claims

1. An assistive rehabilitation system, ARS, comprising a wearable assistive device (A) and an adaptive assistive and/or rehabilitation device, AARD (B),
wherein the wearable assistive device (A) is configured to be supported on the AARD (B) and configured to receive first and second signals indicating moving and storage modes of the AARD (B), respectively, the moving mode being a mode in which the AARD (B) is moveable and at a first height (H1), and the storage mode being a mode in which the AARD (B) is parked such that movement of the AARD (B) is prevented, wherein the moving mode and the storage mode are modes in which a user does not wear the wearable assistive device (A),
wherein the wearable assistive device (A) comprises:
a main frame (4) configured to secure to a waist or a pelvis;
a leg assembly (6) that extends from an end of the main frame (4); and
a main controller (2'), wherein the main controller (2') is configured to control the leg assembly (6) such that the wearable assistive device (A) is spaced apart from a ground upon receiving the first signal indicating the moving mode, and wherein the main controller (2') is configured to control the leg assembly (6) such that the wearable assistive device (A) may contact the ground upon receiving the second signal indicating the storage mode.

2. The assistive rehabilitation system of claim 1, wherein the leg assembly (6) comprises:
an upper leg frame (6a) connected to an end of the main frame (4),
a lower leg frame (6d) connected to an end of the upper leg frame (6a), and
an actuated joint (6b) configured to adjust a knee joint angle (Θ2), wherein the knee joint angle (Θ2) is an angle between the upper leg frame (6a) and the lower leg frame (6d).

3. The assistive rehabilitation system of claim 2, wherein the main controller (2') is configured to reduce a length of the upper leg frame (6a) or a length of the lower leg frame (6d) upon receiving the first signal such that the wearable assistive device (A) is spaced apart from the ground, and wherein the main controller (2') is configured to increase the length of the upper leg frame (6a) or the length of the lower leg frame (6d) upon receiving the second signal such that the wearable assistive device (A) contacts the ground.

4. The assistive rehabilitation system of claim 2 or 3, wherein the main controller (2') is configured to control the actuated joint (6b) to reduce the knee joint angle (Θ2) upon receiving the first signal, and wherein the main controller (2') is configured to control the actuated joint (6b) to increase the knee joint angle (Θ2) upon receiving the second signal.

5. The assistive rehabilitation system of any one of claims 2-4, wherein the main controller (2') is configured to control an actuated hip joint (3) provided between the upper leg frame (6a) and the main frame (4) to reduce a hip joint angle (Θ1) between the upper leg frame (6a) and the main frame (4) upon receiving the first signal, and control the actuated hip joint (3) to increase the hip joint angle (Θ1) upon receiving the second signal.

6. The assistive rehabilitation system of any one of claims 1-5, wherein the first signal is a motion signal that is activated when a wheel of the AARD (B) moves, and the second signal is a braking signal that is activated when a brake is applied to park the AARD (B).

7. The assistive rehabilitation system of any one of claims 1-6, further including:
a foot support (7) provided at an end of the leg assembly (6), the foot support (7) including a pressure sensor for producing a third signal when the foot support (7) contacts the ground;
an actuator provided in at least one joint in the leg assembly (6);
a communication module (2c) provided in the main controller (2') configured to receive the first, second, and third signals; and
a control module (2a) provided in the main controller (2') configured to control the actuator, wherein, when the communication module (2c) receives the first signal and the third signal, the control module (2a) controls the actuator to reduce an angle (Θ1; Θ2) at the at least one joint until the control module (2a) no longer receives the third signal.

8. The assistive rehabilitation system of claim 7, wherein the control module (2a) is configured to control a length of the leg assembly (6), and reduce the length of the leg assembly (6) when the communication module (2c) receives the first signal and the third signal.

9. The assistive rehabilitation system of claim 7, wherein, the control module (2a) is configured to control the actuator to increase the angle (Θ1; Θ2) until the communication module (2c) receives the third signal, when the communication module (2c) receives the second signal and does not receive the third signal; and wherein the control module (2a) is configured to control the actuator to increase the angle (Θ1; Θ2) until the communication module (2c) receives the third signal, when the communication module (2c) does not receive any one of the first, second, or third signals for a predetermined time or more.

10. The assistive rehabilitation system of any one of claims 1-9, wherein the main controller (2') is configured to send an ascending control signal to the AARD (B) to increase the height of the AARD (B), when the main controller (2') receives the first signal and a third signal indicating the wearable assistive device (A) is contacting the ground; and wherein the main controller (2') is configured to send a descending control signal to the AARD (B) to reduce the height of the AARD (B), when the main controller (2') receives the second signal and does not receive the third signal.

11. An assistive rehabilitation system, ARS, comprising a wearable assistive device (A) and an adaptive assistive and/or rehabilitation device, AARD (B),
wherein the wearable assistive device (A) is configured to be supported by the AARD (B) and configured to receive first and second signals indicating a moving mode and a donning mode of the AARD (B), respectively, the moving mode being a mode in which the AARD (B) is moveable and at a standing height (H1), and the donning mode being a mode in which the AARD (B) is parked at a sitting height (H3) such that movement of the AARD (B) is prevented and a seat of the AARD (B) is unfolded, wherein the moving mode is a mode in which a user does not wear the wearable assistive device (A),
wherein the wearable assistive device (A) comprises:
a main frame (4) configured to support a waist;
a leg assembly (6) extending from the main frame (4) and including an actuator provided at a joint;
a foot support (7) provided at an end of the leg assembly (6); and
a main controller (2'), wherein the main controller (2') is configured to control the leg assembly (6) such that the foot support (7) is spaced apart from a ground upon receiving the first signal indicating the moving mode, and wherein the main controller (2') is configured to control the leg assembly (6) such that the wearable assistive device (A) contacts the ground upon receiving the second signal indicating the donning mode.

12. The assistive rehabilitation system of claim 11, wherein the first signal is a motion signal that is activated when a wheel of the AARD (B) moves or a first height signal that is activated when a height sensor (300a) provided in a drive assembly (300) of the AARD (B) senses that the AARD (B) is at the standing height, and the second signal is a second height signal that is activated when the height sensor (300a) of the AARD (B) senses that the AARD (B) is at the sitting height lower than the standing height.

13. The assistive rehabilitation system of claim 11, wherein the first signal is a first height signal that is activated when a position sensor (2b) provided in the main controller (2') senses a position that is equal to or greater than a predetermined standing height, and the second signal is a second height signal that is activated when the position sensor (2b) senses a position that is equal to or less than a predetermined sitting height.

14. The assistive rehabilitation system of any one of claims 11 to 13, wherein the main controller (2') is configured to control a length of the leg assembly (6), and increase the length of the leg assembly (6) upon receiving the second signal so that the foot support (7) contacts the ground.

15. The assistive rehabilitation system of any one of claims 11 to 14, wherein the leg assembly (6) includes two legs that each extend from the main frame (4), wherein the main controller (2') is configured to control a distance between each leg of the leg assembly (6), and increase the distance upon receiving the second signal.

## Patentansprüche

1. Assistierendes Rehabilitationssystem, ARS, aufweisend eine am Körper tragbare Assistenzvorrichtung (A) und eine adaptive Assistenz- und/oder Rehabilitationsvorrichtung, AARD (B),
wobei die tragbare Assistenzvorrichtung (A) konfiguriert ist, an dem AARD (B) gehalten zu werden, und konfiguriert ist, ein erstes und ein zweites Signal zu empfangen, die einen Bewegungs- bzw. Lagerungsmodus der AARD (B) angeben, wobei der Bewegungsmodus ein Modus ist, in dem die AARD (B) bewegbar und in einer ersten Höhe (H1) ist, und der Lagerungsmodus ein Modus ist, in dem die AARD (B) geparkt ist, so dass eine Bewegung der AARD (B) verhindert ist, wobei der Bewegungsmodus und der Lagerungsmodus Modi sind, in denen ein Benutzer die tragbare Assistenzvorrichtung (A) nicht trägt,
wobei die tragbare Assistenzvorrichtung (A) aufweist:
einen Hauptrahmen (4), der konfiguriert ist, um an einer Taille oder einem Becken befestigt zu werden;
eine Beinanordnung (6), die sich von einem Ende des Hauptrahmens (4) erstreckt; und
eine Hauptsteuerung (2'), wobei die Hauptsteuerung (2') konfiguriert ist, die Beinanordnung (6) derart zu steuern, dass beim Empfang des den Bewegungsmodus angebenden ersten Signals die tragbare Assistenzvorrichtung (A) von einem Boden beabstandet wird, und wobei die Hauptsteuerung (2') konfiguriert ist, die Beinanordnung (6) derart zu steuern, dass beim Empfang des den Lagerungsmodus angebenden zweiten Signals die tragbare Assistenzvorrichtung (A) den Boden kontaktieren kann.

2. Assistierendes Rehabilitationssystem nach Anspruch 1, wobei die Beinanordnung (6) aufweist:
einen oberen Beinrahmen (6a), der mit einem Ende des Hauptrahmens (4) verbunden ist,
einen unteren Beinrahmen (6d), der mit einem Ende des oberen Beinrahmens (6a) verbunden ist, und
ein betätigtes Gelenk (6b), das konfiguriert ist, einen Kniegelenkwinkel (02) anzupassen, wobei der Kniegelenkwinkel (θ2) ein Winkel zwischen dem oberen Beinrahmen (6a) und dem unteren Beinrahmen (6d) ist.

3. Assistierendes Rehabilitationssystem nach Anspruch 2, wobei die Hauptsteuerung (2') konfiguriert ist, beim Empfang des ersten Signals eine Länge des oberen Beinrahmens (6a) oder eine Länge des unteren Beinrahmens (6d) zu verringern, so dass die tragbare Assistenzvorrichtung (A) von dem Boden beabstandet wird, und wobei die Hauptsteuerung (2') konfiguriert ist, beim Empfang des zweiten Signals die Länge des oberen Beinrahmens (6a) oder die Länge des unteren Beinrahmens (6d) zu vergrößern, so dass die tragbare Assistenzvorrichtung (A) den Boden kontaktiert.

4. Assistierendes Rehabilitationssystem nach Anspruch 2 oder 3, wobei die Hauptsteuerung (2') konfiguriert ist, das betätigte Gelenk (6b) zu steuern, um beim Empfang des ersten Signals den Kniegelenkwinkel (02) zu verringern, und wobei die Hauptsteuerung (2') konfiguriert ist, das betätigte Gelenk (6b) zu steuern, um beim Empfang des zweiten Signals den Kniegelenkwinkel (02) zu vergrößern.

5. Assistierendes Rehabilitationssystem nach einem der Ansprüche 2 bis 4, wobei die Hauptsteuerung (2') konfiguriert ist, ein betätigtes Hüftgelenk (3), das zwischen dem oberen Beinrahmen (6a) und dem Hauptrahmen (4) bereitgestellt ist, zu steuern, um beim Empfang des ersten Signals einen Hüftgelenkwinkel (01) zwischen dem oberen Beinrahmen (6a) und dem Hauptrahmen (4) zu verringern, und das betätigte Hüftgelenk (3) zu steuern, um beim Empfang des zweiten Signals den Hüftgelenkwinkel (01) zu vergrößern.

6. Assistierendes Rehabilitationssystem nach einem der Ansprüche 1 bis 5, wobei das erste Signal ein Bewegungssignal ist, das aktiviert ist, wenn ein Rad der AARD (B) sich bewegt, und das zweite Signal ein Bremssignal ist, das aktiviert ist, wenn eine Bremse betätigt ist, um die AARD (B) zu parken.

7. Assistierendes Rehabilitationssystem nach einem der Ansprüche 1 bis 6, ferner aufweisend:
eine an einem Ende der Beinanordnung (6) bereitgestellte Fußstütze (7), wobei die Fußstütze (7) einen Drucksensor zum Erzeugen eines dritten Signals, wenn die Fußstütze (7) den Boden kontaktiert, aufweist;
einen Aktuator, der in mindestens einem Gelenk in der Beinanordnung (6) bereitgestellt ist;
ein in der Hauptsteuerung (2') bereitgestelltes Kommunikationsmodul (2c), das konfiguriert ist, das erste, zweite und dritte Signal zu empfangen; und
ein in der Hauptsteuerung (2') bereitgestelltes Steuermodul (2a), das konfiguriert ist, den Aktuator zu steuern,
wobei, wenn das Kommunikationsmodul (2c) das erste Signal und das dritte Signal empfängt, das Steuermodul (2a) den Aktuator steuert, um einen Winkel (θ1; θ2) an dem mindestens einen Gelenk solange zu verringern, bis das Steuermodul (2a) das dritte Signal nicht mehr empfängt.

8. Assistierendes Rehabilitationssystem nach Anspruch 7, wobei das Steuermodul (2a) konfiguriert ist, eine Länge der Beinanordnung (6) zu steuern und die Länge der Beinanordnung (6) zu verringern, wenn das Kommunikationsmodul (2c) das erste Signal und das dritte Signal empfängt.

9. Assistierendes Rehabilitationssystem nach Anspruch 7, wobei das Steuermodul (2a) konfiguriert ist, den Aktuator zu steuern, um, wenn das Kommunikationsmodul (2c) das zweite Signal empfängt und das dritte Signal nicht empfängt, den Winkel (θ1; θ2) solange zu vergrößern, bis das Kommunikationsmodul (2c) das dritte Signal empfängt; und wobei das Steuermodul (2a) konfiguriert ist, den Aktuator zu steuern, um, wenn das Kommunikationsmodul (2c) eines von erstem, zweitem und drittem Signal für eine vorgegebene Zeit oder länger nicht empfängt, den Winkel (θ1; θ2) solange zu vergrößern, bis das Kommunikationsmodul (2c) das dritte Signal empfängt.

10. Assistierendes Rehabilitationssystem nach einem der Ansprüche 1 bis 9, wobei die Hauptsteuerung (2') konfiguriert ist, ein aufsteigendes Steuersignal zu der AARD (B) zu senden, um die Höhe der AARD (B) zu vergrößern, wenn die Hauptsteuerung (2') das erste Signal und ein drittes Signal, das angibt, dass die tragbare Assistenzvorrichtung (A) den Boden kontaktiert, empfängt; und wobei die Hauptsteuerung (2') konfiguriert ist, ein absteigendes Steuersignal zu der AARD (B) zu senden, um die Höhe der AARD (B) zu verringern, wenn die Hauptsteuerung (2') das zweite Signal empfängt und das dritte Signal nicht empfängt.

11. Assistierendes Rehabilitationssystem, ARS, aufweisend eine am Körper tragbare Assistenzvorrichtung (A) und eine adaptive Assistenz- und/oder Rehabilitationsvorrichtung, AARD (B),
wobei die tragbare Assistenzvorrichtung (A) konfiguriert ist, von dem AARD (B) gehalten zu werden, und konfiguriert ist, ein erstes und ein zweites Signal zu empfangen, die einen Bewegungs- bzw. einen Anziehmodus der AARD (B) angeben, wobei der Bewegungsmodus ein Modus ist, in dem die AARD (B) bewegbar und in einer ersten Stehhöhe (H1) ist, und der Anziehmodus ein Modus ist, in dem die AARD (B) auf einer Sitzhöhe (H3) geparkt ist, so dass eine Bewegung der AARD (B) verhindert ist und ein Sitz der AARD (B) entfaltet ist, wobei der Bewegungsmodus ein Modus ist, in dem ein Benutzer die tragbare Assistenzvorrichtung (A) nicht trägt,
wobei die tragbare Assistenzvorrichtung (A) aufweist:
einen Hauptrahmen (4), der konfiguriert ist, eine Taille zu stützen;
eine Beinanordnung (6), die sich von dem Hauptrahmen (4) erstreckt und einen an einem Gelenk bereitgestellten Aktuator aufweist;
eine Fußstütze (7), die an einem Ende der Beinanordnung (6) bereitgestellt ist; und
eine Hauptsteuerung (2'), wobei die Hauptsteuerung (2') konfiguriert ist, die Beinanordnung (6) derart zu steuern, dass beim Empfang des den Bewegungsmodus angebenden ersten Signals die Fußstütze (7) von einem Boden beabstandet wird, und wobei die Hauptsteuerung (2') konfiguriert ist, die Beinanordnung (6) derart zu steuern, dass beim Empfang des den Anziehmodus angebenden zweiten Signals die tragbare Assistenzvorrichtung (A) den Boden kontaktiert.

12. Assistierendes Rehabilitationssystem nach Anspruch 11, wobei das erste Signal ein Bewegungssignal ist, das aktiviert ist, wenn ein Rad der AARD (B) sich bewegt, oder ein Erste-Höhe-Signal ist, das aktiviert ist, wenn ein in einer Antriebsanordnung (300) der AARD (B) bereitgestellter Höhensensor (300a) detektiert, dass die AARD (B) in einer Stehhöhe ist, und das zweite Signal ein Zweite-Höhe-Signal ist, das aktiviert ist, wenn der Höhensensor (300a) der AARD (B) detektiert, dass die AARD (B) in einer Sitzhöhe ist, die niedriger als die Stehhöhe ist.

13. Assistierendes Rehabilitationssystem nach Anspruch 11, wobei das erste Signal ein Erste-Höhe-Signal ist, das aktiviert ist, wenn ein in der Hauptsteuerung (2') bereitgestellter Positionssensor (2b) eine Position detektiert, die gleich oder größer als eine vorgegebene Stehhöhe ist, und das zweite Signal ein Zweite-Höhe-Signal ist, das aktiviert ist, wenn der Positionssensor (2b) eine Position detektiert, die gleich oder weniger als eine vorgegebene Sitzhöhe ist.

14. Assistierendes Rehabilitationssystem nach einem der Ansprüche 11 bis 13, wobei die Hauptsteuerung (2') konfiguriert ist, eine Länge der Beinanordnung (6) zu steuern und beim Empfang des zweiten Signals die Länge der Beinanordnung (6) zu vergrößern, so dass die Fußstütze (7) den Boden kontaktiert.

15. Assistierendes Rehabilitationssystem nach einem der Ansprüche 11 bis 14, wobei die Beinanordnung (6) zwei Beine aufweist, die sich jeweils von dem Hauptrahmen (4) erstrecken, wobei die Hauptsteuerung (2') konfiguriert ist, einen Abstand zwischen jedem Bein der Beinanordnung (6) zu steuern und beim Empfang des zweiten Signals den Abstand zu vergrößern.

## Revendications

1. Système d'assistance à la rééducation, ARS, comprenant un dispositif d'assistance portable (A) et un dispositif d'assistance et/ou de rééducation adaptatif, AARD (B), dans lequel le dispositif d'assistance portable (A) est configuré pour être supporté sur l'AARD (B) et configuré pour recevoir des premier et deuxième signaux indiquant des modes de déplacement et de stockage de l'AARD (B), respectivement, le mode de déplacement étant un mode dans lequel l'AARD (B) est mobile et à une première hauteur (H1), et le mode de stockage étant un mode dans lequel l'AARD (B) est parqué de sorte qu'un déplacement de l'AARD (B) soit empêché, dans lequel le mode de déplacement et le mode de stockage sont des modes dans lesquels un utilisateur ne porte pas le dispositif d'assistance portable (A),
dans lequel le dispositif d'assistance portable (A) comprend :
un cadre principal (4) configuré pour se fixer à une taille ou à un bassin ;
un ensemble de jambe (6) qui s'étend depuis une extrémité du cadre principal (4) ; et un dispositif de commande principal (2'), dans lequel le dispositif de commande principal (2') est configuré pour commander l'ensemble de jambe (6) de sorte que le dispositif d'assistance portable (A) soit espacé du sol lors de la réception du premier signal indiquant le mode de déplacement, et dans lequel le dispositif de commande principal (2') est configuré pour commander l'ensemble de jambe (6) de sorte que le dispositif d'assistance portable (A) puisse entrer en contact avec le sol lors de la réception du deuxième signal indiquant le mode de stockage.

2. Système d'assistance à la rééducation selon la revendication 1, dans lequel l'ensemble de jambe (6) comprend :
un cadre de jambe supérieur (6a) connecté à une extrémité du cadre principal (4),
un cadre de jambe inférieur (6d) connecté à une extrémité du cadre de jambe supérieur (6a), et
une articulation actionnée (6b) configurée pour régler un angle d'articulation du genou (02), dans lequel l'angle d'articulation du genou (02) est un angle entre le cadre de jambe supérieur (6a) et le cadre de jambe inférieur (6d).

3. Système d'assistance à la rééducation selon la revendication 2, dans lequel le dispositif de commande principal (2') est configuré pour réduire une longueur du cadre de jambe supérieur (6a) ou une longueur du cadre de jambe inférieur (6d) lors de la réception du premier signal, de sorte que le dispositif d'assistance portable (A) soit espacé du sol, et dans lequel le dispositif de commande principal (2') est configuré pour augmenter la longueur du cadre de jambe supérieur (6a) ou la longueur du cadre de jambe inférieur (6d) lors de la réception du deuxième signal, de sorte que le dispositif d'assistance portable (A) entre en contact avec le sol.

4. Système d'assistance à la rééducation selon la revendication 2 ou 3, dans lequel le dispositif de commande principal (2') est configuré pour commander l'articulation actionnée (6b) pour réduire l'angle d'articulation du genou (θ2) lors de la réception du premier signal, et dans lequel le dispositif de commande principal (2') est configuré pour commander l'articulation actionnée (6b) pour augmenter l'angle d'articulation du genou (02) lors de la réception du deuxième signal.

5. Système d'assistance à la rééducation selon l'une quelconque des revendications 2 à 4, dans lequel le dispositif de commande principal (2') est configuré pour commander une articulation de hanche actionnée (3) prévue entre le cadre de jambe supérieur (6a) et le cadre principal (4) pour réduire un angle d'articulation de hanche (01) entre le cadre de jambe supérieur (6a) et le cadre principal (4) lors de la réception du premier signal, et commander l'articulation de hanche actionnée (3) pour augmenter l'angle d'articulation de hanche (01) lors de la réception du deuxième signal.

6. Système d'assistance à la rééducation selon l'une quelconque des revendications 1 à 5, dans lequel le premier signal est un signal de mouvement qui est activé lorsqu'une roue de l'AARD (B) se déplace, et le deuxième signal est un signal de freinage qui est activé lorsqu'un frein est appliqué pour parquer l'AARD (B).

7. Système d'assistance à la rééducation selon l'une quelconque des revendications 1 à 6, comportant en outre :
un support de pied (7) prévu à une extrémité de l'ensemble de jambe (6), le support de pied (7) comportant un capteur de pression pour produire un troisième signal lorsque le support de pied (7) entre en contact avec le sol ;
un actionneur prévu dans au moins une articulation de l'ensemble de jambe (6) ;
un module de communication (2c) prévu dans le dispositif de commande principal (2') configuré pour recevoir les premier, deuxième et troisième signaux ; et
un module de commande (2a) prévu dans le dispositif de commande principal (2') configuré pour commander l'actionneur, dans lequel, lorsque le module de communication (2c) reçoit le premier signal et le troisième signal, le module de commande (2a) commande l'actionneur pour réduire un angle (θ1 ; θ2) au niveau de l'au moins une articulation jusqu'à ce que le module de commande (2a) ne reçoive plus le troisième signal.

8. Système d'assistance à la rééducation selon la revendication 7, dans lequel le module de commande (2a) est configuré pour commander une longueur de l'ensemble de jambe (6), et réduire la longueur de l'ensemble de jambe (6) lorsque le module de communication (2c) reçoit le premier signal et le troisième signal.

9. Système d'assistance à la rééducation selon la revendication 7, dans lequel le module de commande (2a) est configuré pour commander l'actionneur pour augmenter l'angle (θ1 ; θ2) jusqu'à ce que le module de communication (2c) reçoive le troisième signal, lorsque le module de communication (2c) reçoit le deuxième signal et ne reçoit pas le troisième signal ; et dans lequel le module de commande (2a) est configuré pour commander l'actionneur pour augmenter l'angle (θ1 ; θ2) jusqu'à ce que le module de communication (2c) reçoive le troisième signal, lorsque le module de communication (2c) ne reçoit aucun des premier, deuxième ou troisièmes signaux pendant un temps prédéterminé ou plus.

10. Système d'assistance à la rééducation selon l'une quelconque des revendications 1 à 9, dans lequel le dispositif de commande principal (2') est configuré pour envoyer un signal de commande ascendant à l'AARD (B) pour augmenter la hauteur de l'AARD (B), lorsque le dispositif de commande principal (2') reçoit le premier signal et un troisième signal indiquant que le dispositif d'assistance portable (A) est en contact avec le sol ; et dans lequel le dispositif de commande principal (2') est configuré pour envoyer un signal de commande descendant à l'AARD (B) pour réduire la hauteur de l'AARD (B), lorsque le dispositif de commande principal (2') reçoit le deuxième signal et ne reçoit pas le troisième signal.

11. Système d'assistance à la rééducation, ARS, comprenant un dispositif d'assistance portable (A) et un dispositif d'assistance et/ou de rééducation adaptatif, AARD (B), dans lequel le dispositif d'assistance portable (A) est configuré pour être supporté par l'AARD (B) et configuré pour recevoir des premier et deuxième signaux indiquant un mode de déplacement et un mode d'enfilage de l'AARD (B), respectivement, le mode de déplacement étant un mode dans lequel l'AARD (B) est mobile et à une hauteur (H1) debout, et le mode d'enfilage étant un mode dans lequel l'AARD (B) est parqué à une hauteur (H3) assise de sorte qu'un déplacement de l'AARD (B) soit empêché et qu'un siège de l'AARD (B) soit déplié, dans lequel le mode de déplacement est un mode dans lequel un utilisateur ne porte pas le dispositif d'assistance portable (A), dans lequel le dispositif d'assistance portable (A) comprend :
un cadre principal (4) configuré pour supporter une taille ;
un ensemble de jambe (6) s'étendant depuis le cadre principal (4) et comportant un actionneur prévu au niveau d'une articulation ;
un support de pied (7) prévu à une extrémité de l'ensemble de jambe (6) ; et
un dispositif de commande principal (2'), dans lequel le dispositif de commande principal (2') est configuré pour commander l'ensemble de jambe (6) de sorte que le support de pied (7) soit espacé du sol lors de la réception du premier signal indiquant le mode de déplacement, et dans lequel le dispositif de commande principal (2') est configuré pour commander l'ensemble de jambe (6) de sorte que le dispositif d'assistance portable (A) entre en contact avec le sol lors de la réception du deuxième signal indiquant le mode d'enfilage.

12. Système d'assistance à la rééducation selon la revendication 11, dans lequel le premier signal est un signal de mouvement qui est activé lorsqu'une roue de l'AARD (B) se déplace, ou un premier signal de hauteur qui est activé lorsqu'un capteur de hauteur (300a) prévu dans un ensemble d'entraînement (300) de l'AARD (B) détecte que l'AARD (B) est à la hauteur debout, et le deuxième signal est un deuxième signal de hauteur qui est activé lorsque le capteur de hauteur (300a) de l'AARD (B) détecte que l'AARD (B) est à la hauteur assise inférieure à la hauteur debout.

13. Système d'assistance à la rééducation selon la revendication 11, dans lequel le premier signal est un premier signal de hauteur qui est activé lorsqu'un capteur de position (2b) prévu dans le dispositif de commande principal (2') détecte une position qui est égale ou supérieure à une hauteur debout prédéterminée, et le deuxième signal est un deuxième signal de hauteur qui est activé lorsque le capteur de position (2b) détecte une position qui est égale ou inférieure à une hauteur assise prédéterminée.

14. Système d'assistance à la rééducation selon l'une quelconque des revendications 11 à 13, dans lequel le dispositif de commande principal (2') est configuré pour commander une longueur de l'ensemble de jambe (6), et augmenter la longueur de l'ensemble de jambe (6) lors de la réception du deuxième signal, de sorte que le support de pied (7) entre en contact avec le sol.

15. Système d'assistance à la rééducation selon l'une quelconque des revendications 11 à 14, dans lequel l'ensemble de jambe (6) comporte deux jambes qui s'étendent chacune depuis le cadre principal (4), dans lequel le dispositif de commande principal (2') est configuré pour commander une distance entre chaque jambe de l'ensemble de jambe (6), et augmenter la distance lors de la réception du deuxième signal.
